(19) **Europäisches Patentamt / European Patent Office / Office européen des brevets**

(11) **EP 2 929 348 B1**

(12) # EUROPEAN PATENT SPECIFICATION

(45) Date of publication and mention of the grant of the patent:
**10.05.2017 Bulletin 2017/19**

(21) Application number: **13799325.9**

(22) Date of filing: **05.12.2013**

(51) Int Cl.:
***G01N 33/50*** *(2006.01)*   ***G01N 33/68*** *(2006.01)*

(86) International application number:
**PCT/EP2013/075643**

(87) International publication number:
**WO 2014/086916 (12.06.2014 Gazette 2014/24)**

(54) **ANTI-MIF ANTIBODY CELL MIGRATION ASSAY**

ZELLWANDERUNGSASSAY FÜR ANTI-MIF-ANTIKÖRPER

ESSAI DE MIGRATION CELLULAIRE AVEC ANTICORPS ANTI-MIF

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **07.12.2012 US 201261734845 P**
**12.03.2013 US 201361778141 P**

(43) Date of publication of application:
**14.10.2015 Bulletin 2015/42**

(73) Proprietors:
• **Baxalta GmbH**
**8152 Glattpark, Opfikon (CH)**
• **Baxalta Incorporated**
**Bannockburn, IL 60015 (US)**

(72) Inventors:
• **THIELE, Michael**
**1030 Wien (AT)**
• **KERSCHBAUMER, Randolf**
**3400 Klosterneuburg (AT)**

(74) Representative: **Hoffmann Eitle**
**Patent- und Rechtsanwälte PartmbB**
**Arabellastraße 30**
**81925 München (DE)**

(56) References cited:
**EP-A1- 2 231 707**

• **H. FAN ET AL: "Macrophage Migration Inhibitory Factor and CD74 Regulate Macrophage Chemotactic Responses via MAPK and Rho GTPase", THE JOURNAL OF IMMUNOLOGY, vol. 186, no. 8, 16 March 2011 (2011-03-16), pages 4915-4924, XP055097776, ISSN: 0022-1767, DOI: 10.4049/jimmunol.1003713**
• **HAGEMANN THORSTEN ET AL: "Macrophages induce invasiveness of epithelial cancer cells via NF-kappa B and JNK", THE JOURNAL OF IMMUNOLOGY, THE AMERICAN ASSOCIATION OF IMMUNOLOGISTS, US, vol. 175, no. 2, 1 July 2005 (2005-07-01), pages 1197-1205, XP002463041, ISSN: 0022-1767**
• **GERRIT GRIEB ET AL: "Macrophage migration inhibitory factor is a potential inducer of endothelial progenitor cell mobilization after flap operation", SURGERY, vol. 151, no. 2, 1 February 2012 (2012-02-01), pages 268-277.e1, XP055099121, ISSN: 0039-6060, DOI: 10.1016/j.surg.2010.10.008**

EP 2 929 348 B1

**Description**

The present invention pertains to an assay which is suitable to test the potency of anti-MIF antibodies. In particular, the present invention is directed to an advantageous and easy-to-use cell migration assay.

**BACKGROUND**

Macrophage migration inhibitory factor (MIF) is a cytokine initially isolated based upon its ability to inhibit the *in vitro* random migration of peritoneal exudate cells from tuberculin hypersensitive guinea pigs (containing macrophages) (Bloom et al. Science 1966,153,80-2; David et al. PNAS 1966,56,72-7). Today, MIF is known as a critical upstream regulator of the innate and acquired immune response that exerts a pleiotropic spectrum of activities.

The human MIF cDNA was cloned in 1989 (Weiser et al., PNAS 1989, 86, 7522-6), and its genomic localization was mapped to chromosome 22. The product of the human MIF gene is a protein with 114 amino acids (after cleavage of the N-terminal methionine) and an apparent molecular mass of about 12.5 kDa. MIF has no significant sequence homology to any other protein. The protein crystallizes as a trimer of identical subunits. Each monomer contains two antiparallel alpha-helices that pack against a four-stranded beta-sheet. The monomer has additional two beta-strands that interact with the beta-sheets of adjacent subunits to form the interface between monomers. The three subunits are arranged to form a barrel containing a solvent-accessible channel that runs through the center of the protein along a molecular three-fold axis (Sun et al. PNAS 1996, 93, 5191-5196).

It was reported that MIF secretion from macrophages was induced at very low concentrations of glucocorticoids (Calandra et al. Nature 1995, 377, 68-71). However, MIF also counter-regulates the effects of glucocorticoids and stimulates the secretion of other cytokines such as tumor necrosis factor TNF-$\alpha$ and interleukin IL-1 ß (Baugh et al., Crit Care Med 2002, 30, S27-35). MIF was also shown e.g. to exhibit pro-angiogenic, pro-proliferative and anti-apoptotic properties, thereby promoting tumor cell growth (Mitchell, R.A., Cellular Signalling, 2004.16(1): p. 13-19; Lue, H. et al., Oncogene 2007.26(35): p. 5046-59). It is also e.g. directly associated with the growth of lymphoma, melanoma, and colon cancer (Nishihira et al. J Interferon Cytokine Res. 2000, 20:751-62).

MIF is a mediator of many pathologic conditions and thus associated with a variety of diseases including *inter alia* inflammatory bowel disease (IBD), rheumatoid arthritis (RA), acute respiratory distress syndrome (ARDS), asthma, glomerulonephritis, IgA nephropathy, myocardial infarction (MI), sepsis and cancer, though not limited thereto.

Polyclonal and monoclonal anti-MIF antibodies have been developed against recombinant human MIF (Shimizu et al., FEBS Lett. 1996; 381,199-202; Kawaguchi et al, Leukoc. Biol. 1986, 39, 223-232, and Weiser et al., Cell. Immunol. 1985, 90, 167-78).

Anti-MIF antibodies have been suggested for therapeutic use. Calandra et al., (J. Inflamm. (1995), 47, 39-51) reportedly used anti-MIF antibodies to protect animals from experimentally induced gram-negative and gram-positive septic shock. Anti-MIF antibodies were suggested as a means of therapy to modulate cytokine production in septic shock and other inflammatory disease states.

US 6,645,493 discloses monoclonal anti-MIF antibodies derived from hybridoma cells, which neutralize the biological activity of MIF. It could be shown in an animal model that these mouse-derived anti-MIF antibodies had a beneficial effect in the treatment of endotoxin-induced shock.

US 200310235584 discloses methods of preparing high affinity antibodies to MIF in animals in which the MIF gene has been homozygously knocked-out.

From EP 2 231 707 describes an assay for MIF. This assay uses exogenously added MIF and thus differs from the presently provided method.

From Hagemann et al., Journal of Immunology, The American Association of Immunologists, US, Vol. 175, No. 2, July 1 2005, pages 1197-1205 it was known that macrophages induce invasiveness of epithelial cancer cells via NF-$\kappa$B and JNK. However, this document only provides an assay where test cells are tested for chemotaxis and does not provide for determination of the potency of anti-(ox)MIF antibodies.

Glycosylation-inhibiting factor (GIF) is a protein described by Galat et al. (Eur. J. Biochem, 1994, 224, 417-21). MIF and GIF are now recognized to be identical. Watarai et al. (PNAS 2000, 97, 13251-6) described polyclonal antibodies binding to different GIF epitopes to identify the biochemical nature of the posttranslational modification of GIF in Ts cells. Watarai et al, *supra*, reported that GIF occurs in different conformational isoforms *in vitro*. One type of isomer occurs by chemical modification of a single cysteine residue. The chemical modification leads to conformational changes within the GIF protein.

In order to test the potency of anti-MIF antibodies a reliable and easy-to-use assay needs to be provided. It is of paramount importance, both for diagnostic and therapeutic purposes, to be able to define a given anti-MIF antibody-sample as to its potency. Without a clear indication of the respective potency of an anti-MIF antibody, the same cannot be used for either diagnosis or therapy.

Robust bioassays for potency assessment of MIF related drugs, in particular of anti-MIF antibodies, are not known so far.

Although it was shown in earlier assay formats that the MIF protein exhibits chemokine-like functions and that these functions can be blocked by anti-MIF antibodies (e.g. Bemhagen et al., Nature Medicine, 2007), no robust MIF bioassay based on the inhibition of autocrine-induced cell migration of e.g. monocytic cells has been established and qualified so far.

Thus, the object of the present invention is the provision of an assay which can determine the potency of anti-MIF antibodies. Preferably, this assay and respective assay method should be capable of providing highly sensitive and reproducible results and should at the same time be easy-to-use.

The present inventors set out to investigate how this goal could be achieved and have thus accomplished the present invention.

## SUMMARY OF THE INVENTION

The present invention is directed to a highly sensitive, reproducible and easy to use - assay to determine the potency of anti-MIF antibodies, in particular anti-oxMIF antibodies.

The inventive assay is a cell migration assay, which is defined as follows:

1. Assay method for determining the potency of anti-MIF antibodies, wherein a cell migration is performed, wherein the assay method comprises the following steps:

- providing cells, which are capable of migration, in a first part of a device, wherein the cells express MIF,
- adding the anti-(ox)MIF antibody to be determined to a second part of the device, which is configured to be in a connection with the first part of the device which allows cell migration and diffusion,
- calculating inhibition of migration, and wherein no(ox)MIF is added to the device. "Potency" in this context is a measure of drug activity expressed in terms of the amount required to produce an effect of given intensity. It is preferably expressed as an $IC_{50}$ value (half maximal inhibition).

The assay of the present invention is based on the principle of inhibiting autocrine chemotactic actions of (ox)MIF and thereby inhibiting migration of cells which express MIF, in particular oxMIF, preferably on their surface, e.g. certain monocytic cells, like U937 monocytic cells. The cells of the present invention can express MIF endogenously or can be manipulated to recombinantly express MIF. The MIF needs to be expressed as oxMIF/converted to oxMIF. In a preferred embodiment the oxMIF is expressed on the surface of the cells, though this is not necessarily a key feature of this invention.

The present assay is to be differentiated from a typical chemotaxis assay (as referred to in scientific literature) which is based on a directed migration of cells towards a chemoattractant gradient. This chemoattractant gradient according to the prior art would have been (ox)MIF, added to the device in addition to the cells. The present inventors could surprisingly show that it is possible to provide an assay using cells which express MIF and that it was thus possible to provide an assay without addition of a further chemoattractant, i.e. without addition of (ox) MIF. These findings were particularly advantageous as the resultant assay is ox-MIF-diffusion independent, i.e. diffusion over time will not interfere with the results, and thus the assay is not as time-sensitive as prior art assays. Anti-(ox)MIF antibodies are capable of inhibiting the pro-migratory (ox)MIF functions on cells expressing (ox)MIF, thereby inhibiting random cell migration ("chemokinesis") (rather than inhibiting cell chemotaxis as referred to in scientific literature).

The present invention is also further explained by the attached figures.

Figure 1: General set-up of the present assay method.
Figure 2: FACS data of oxMIF on U937 monocytic cells

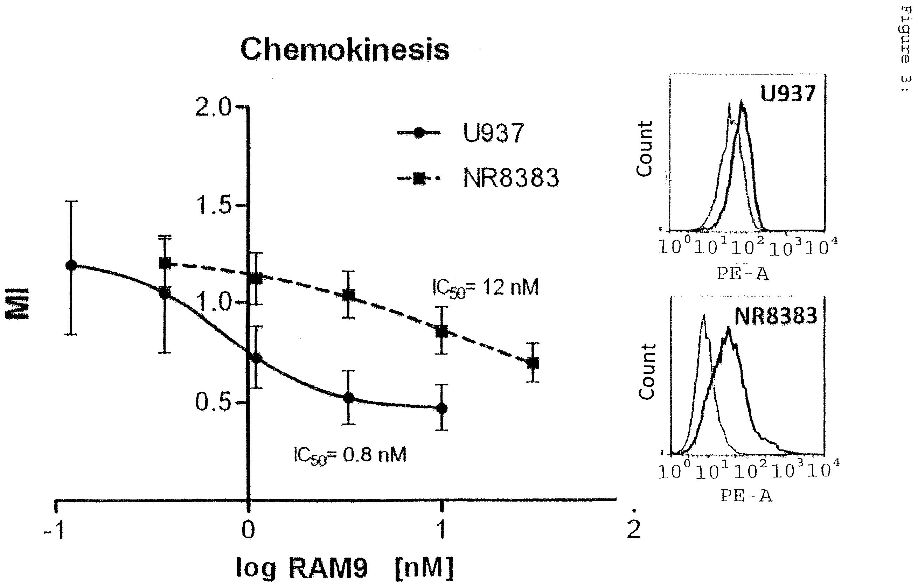

Figure 3: Exemplary figure showing two migration inhibition curves of U937 (human monocytes) and NR8383 (rat macrophages) towards different concentrations of RAM9 (anti-oxMIF antibody; logarithmic scale). FACS plot shows the binding of RAM9 to the cellular surface of these cells (black line; thin line: isotype control antibody)

## DETAILED DESCRIPTION OF THE INVENTION

The invention is characterized, in part, by the following items:

1. Assay method for determining the potency of anti-MIF antibodies, wherein a cell migration is performed, wherein the assay method comprises the following steps:

- providing cells, which are capable of migration, in a first part of a device, wherein the cells express MIF,

- adding the anti-(ox)MIF antibody to be determined to a second part of the device, which is configured to be in a connection with the first part of the device which allows cell migration and diffusion,
- calculating inhibition of migration, and wherein no(ox)MIF is added to the device.

2. Assay method according to item 1, wherein the anti-MIF antibodies are anti-oxMIF antibodies, preferably wherein the antibodies are selected from the group consisting of RAB0, RAB4, RAB9, RAM0, RAM4 and/or RAM9, very preferred RAM9.

3. Assay method according to item 1 or 2, wherein the device comprises an upper and a lower chamber, which are connected via a separating membrane with pores and in that the cells are added to the upper chamber and in that the antibodies are added to the lower chamber, preferably wherein the device is a two chamber cell migration assay (modified Boyden chamber assay), more preferably a Transwell cell migration assay device.

In a different embodiment, the device comprises an upper and a lower chamber, which are connected via a separating membrane with pores and wherein both the cells and the antibodies are added to the upper chamber, preferably wherein the device is a two chamber cell migration assay (modified Boyden chamber assay), more preferably a Transwell cell migration assay device.

In both cases, the number of migrated cells will be measured in the lower chamber.

4. Assay method according to any one of the preceding items, wherein the cells are cells which express (ox)MIF, preferably on their cell surface, preferably wherein the cells are cells from disease samples, more preferred wherein the cells are monocytic cells, preferably human monocytic cells, more preferred human immortalized monocytic cells, most preferred U937 cells, or THP-1 human monocytic cells or in an alternative embodiment rat NR 8383 monocytic cells.

5. Assay method according to any one of the preceding items, wherein the cells are provided as a cell suspension in a suitable migration medium to allow migration.

6. Assay method according to item 5, wherein the migration medium does not comprise proteins.

7. Assay method according to any one of the preceding items, wherein the antibody is added in a non toxic biological buffer with a moderately weak acid and its conjugate base, preferably a glycine buffer, an N-substituted taurin buffer, or a phosphate buffer saline (PBS) buffer, to the second part of e.g. the Transwell® chamber.

In a preferred embodiment of the assay, the concentration of the antibody to be tested is determined on the basis of routine dilution experiments. In a first step, the expected $IC_{50}$ would be determined, wherein the $IC_{50}$ value should be in the same range as the $K_D$ value (affinity constant). Typically, a dilution series having the antibody in several, e.g. 6 - 10, concentrations between 0.01 and 100 nM would be established, thus determining the expected $IC_{50}$ value. In the actual assay, this expected $IC_{50}$ would then be used as the fixed middle point of a dilution series, which would typically have up to 5 concentration steps below the $IC_{50}$ value and up to 5 concentration steps above the $IC_{50}$ value. The concentration step would typically increase and decrease, respectively, exponentially, e.g. with a three, four or five exponent.

Thus, assuming that the expected $IC_{50}$ as determined in the preliminary experiment was 1 nM, the concentrations actually employed in the assay would be (using 5 as an exponent and 3 steps): 0.008, 0.04, 0.2, 1, 5, 25 and 125 nM.

8. Assay method according to any one of the preceding items, wherein the antibody is added to have a final concentration in the assay of 0.01 - 100 nM, preferably 0.02 to 50 nM, preferably 0.04-30 nM, preferably as a dilution series.

In a preferred embodiment of the present assay method, the assay is incubated for a time period until a suitable dose response curve is observed, as can be determined by the person skilled in the art. This incubation period will vary depending on the cells which are used in the assay; it will also vary according to the pore size used for the membrane in the assay chamber. Larger pore sizes will lead to a more rapid distribution of the cells and thus shorter incubation periods, but might lead to unspecific results. The adjustment of the incubation time can be done in a preliminary experiment and would be well within the general skill of a person skilled in the art.

9. Assay method according to any one of the preceding items, wherein the assay, e.g. the Transwell® chamber, is incubated after addition of the cells and the antibody for 6-20 h, preferably 8-12 h, preferably at approximately 37°C, wherein the cells are U937 cells, and wherein the membrane has a pore size of approximately 5 μm.

10. Assay method according to any one of the preceding items, wherein the migrated cells are counted, preferably after the above incubation step, and preferably in the lower chamber, whereupon information about the potency of the tested antibody can be obtained, preferably by calculating the half-maximal inhibiting antibody concentration ($IC_{50}$-value).

11. Assay method according to any one of the preceding items, wherein the cells undergo from 10 to 48 h, preferably 8-16, preferably 10-12 h serum starvation before they are used in the assay.

"Serum starvation" in this context shall mean that the cells have been cultured for the time as indicated above in a medium which is free of serum, preferably free of fetal bovine serum. The serum starvation needs to be carried out to make sure that no serum components are included into the actual assay method. If serum components were comprised in the actual assay method, the results could become unspecific as serum components are known to act as potent chemoattractants (serum is also used as a positive control in the example below).

12. Assay method according to any one of the preceding items, wherein the cells are derived from a working bank.

13. Assay method according to any one of the preceding items, wherein no (ox)MIF is added to the device.

This obviates the need for the preparation of recombinant MIF protein and thus provides for one of the advantages of the present assay.

The present assay method can be carried out with a cell number which can be determined by the person skilled in the art based on his or her general knowledge

This cell number is defined as being the number of cells as added to each well (preferably upper chamber) of the respective assay device. Reference aspects pertain to:

14. Assay kit for determining the potency of anti-(ox)MIF-antibodies, comprising all reagents necessary to carry out the assay method of any one or more of the preceding items, preferably

- cells which express MIF
- antibody dilution buffer (e.g. glycine buffer)
- migration medium, and/or
- two chamber cell migration system.

The preferred components of this kit will be explained in more detail below.

15. Pharmaceutical or diagnostic composition, comprising anti-(ox)MIF antibodies, wherein the anti-(ox)MIF antibodies are characterized in that they have undergone a potency determination, as defined in any one of the precedings claims.

According to the invention, it is for the first time possible to reliably provide a pharmaceutical and diagnostic composition which comprises anti-(ox) MIF antibodies, in particular RAM0, RAM4, RAM0, RAB9, RAB0 and/or RAB4, most preferred RAM9, wherein the antibodies are defined with regard to their potency. The resultant antibody formulation will be consistent as to the antibodies' potency.

Elevated MIF levels, i.e. levels of MIF in general are detected after the onset of various diseases, *inter alia* after the onset of cancer. However, MIF circulates also in healthy subjects, which makes a clear differentiation difficult. oxMIF, on the contrary, is not present in healthy subjects and therefore is a much stronger diagnostic marker for MIF-related diseases. As has been shown in earlier work of the present inventors, oxMIF is increased in disease states and detectable in samples of patients, like e.g. plasma, blood, serum and urine.

Baxter antibodies RAB9, RAB4 and RAB0, as well as RAM9, RAM4, and RAM0, respectively, specifically bind to oxMIF (and are incapable of binding to redMIF).

In earlier experiments carried out by the inventors, it could be shown that oxidative procedures like cystine-mediated oxidation, GSSG (ox. Glutathione)-mediated oxidation or incubation of MIF with Proclin300 or protein crosslinkers (e.g. BMOE) causes binding to the above mentioned antibodies.

The surprising conclusions reached by the present inventors are:

* Redox modulation (Cys/Glu-mediated mild oxidation) of recombinant MIF (human, murine, rat, CHO, monkey)) or treatment of recombinant MIF with Proclin300 or protein crosslinkers leads to the binding of Baxter's anta-MIF antibodies RAB9, RAB4 and RAB0
* Reduction of oxMIF leads to the loss of Ab binding
* Specificity for oxMIF-isoforms correlates with biological Ab efficacy (*in vitro/in vivo*).
* Only oxMIF levels, but not necessarily MIF levels, are correlated with a disease state.

The above mentioned antibodies are characterized and supported by both their sequences as well as by deposits as plasmids in *E.coli* (strain TG1), comprising either the light or the heavy chain of each of the above mentioned antibodies RAB0, RAB4 and RAB9, respectively as well as of RAM0, RAM4 and RAM9.

The plasmids are characterized by their DSM number which is the official number as obtained upon deposit under the Budapest Treaty with the German Collection of Microorganisms and Cell Cultures (DSMZ), Mascheroder Weg 1 b, Braunschweig, Germany. The plasmids were deposited in *E. coli* strains, respectively. The plasmid with the DSM 25110 number comprises the light chain sequence of the anti-MIF antibody RAB4. The plasmid with the DSM 25112 number comprises the heavy chain (IgG4) sequence of the anti-MIF antibody RAB4.

The co-expression of plasmids DSM 25110 and DSM 25112 in a suitable host cell results in the production of preferred anti-MIF antibody RAB4.

The plasmid with the DSM 25111 number comprises the light chain sequence of the anti-MIF antibody RAB9. The plasmid with the DSM 25113 number comprises the heavy chain (IgG4) sequence of the anti-MIF antibody RAB9.

The co-expression of plasmids DSM 25111 and DSM 25113 in a suitable host cell results in the production of preferred anti-MIF antibody RAB9.

The plasmid with the DSM 25114 number comprises the light chain sequence of the anti-MIF antibody RAB0. The plasmid with the DSM 25115 number comprises the heavy chain (IgG4) sequence of the anti-MIF antibody RAB0.

The co-expression of plasmids DSM 25114 and DSM 25115 in a suitable host cell results in the production of preferred anti-MIF antibody RAB0.

Also deposited are antibodies RAM0, RAM9 and RAM4; all have been deposited with the DSMZ, Braunschweig, Germany on April

12,2012 according to the Budapest Treaty, with the

following designations:

> RAM9 - heavy chain: E.coli GA.662-01.pRAM9hc - DSM 25860.
> RAM4 - light chain: E.coli GA.906-04.pRAM4lc - DSM 25861.
> RAM9 - light chain: E.coli GA.661-01.pRAM9lc - DSM 25859.
> RAM4 - heavy chain: E.coli GA.657-02.pRAM4hc - DSM 25862.
> RAM0 - light chain: E.coli GA.906-01.pRAM0lc - DSM 25863.
> RAM0 - heavy chain: E.coli GA.784-01.pRAM0hc - DSM 25864.

A biological sample in the context of this application is preferably a body fluid sample of the subject on which/whom the diagnosis shall be performed or a tissue or cell sample. A body fluid sample is any sample of a body fluid as known to a person skilled in the art. Exemplary, but not limiting, such a sample can be blood, plasma, serum, saliva, urine, nasal fluid, ascites, ocular fluid, amniotic fluid, aqueous humour, vitreous humour, tear fluid, Cowper's fluid, semen, interstitial fluid, lymph, breast milk, mucus (incl. snot and phlegm), pleural fluid, pus, menses, vaginal lubrication, sebum, cerebrospinal fluid and synovial fluid. Further biological samples in the context of this application can be lavages (washing outs) of a (hollow) body organ (e.g. bronchoalveolar lavage, stomach lavage and bowel lavage).A tissue sample is preferably a tissue biopsy, e.g. a needle core biopsy.

A biological sample in the context of this application in an alternative embodiment, is a cell sample, most preferably a cell sample from the circulation or the diseased tissue, more preferably as a single cell suspension sample, of the subject on which the diagnosis shall be performed.

The term "prophylactic" or "therapeutic" treatment is art-recognized and refers to administration of a drug to a patient. If it is administered prior to clinical manifestation of the unwanted condition (e.g. disease or other unwanted state of the host, e.g. a human or an animal) then the treatment is prophylactic, i.e., it protects the host against developing the unwanted condition, whereas if administered after manifestation of the unwanted condition, the treatment is therapeutic (i.e., it is intended to diminish, ameliorate or maintain the existing unwanted condition or side effects thereof).

As used herein an anti-(ox)MIF compound refers to any agent that attenuates, inhibits, opposes, counteracts, or decreases the biological activity of (ox)MIF. An anti(ox)MIF compound may be an agent that inhibits or neutralizes (ox)MIF activity, for example an antibody, particularly preferred, the antibodies as described herein, even more preferred the antibodies RAB9, RAB4 and/or RAB0, or RAM9, RAM4 and/or RAM0, respectively.

## Definitions and General Techniques

Unless otherwise defined herein, scientific and technical terms used in connection with the present invention shall have the meanings that are commonly understood by those of ordinary skill in the art. Generally, nomenclatures used in connection with, and techniques of, cell and tissue culture, molecular biology, immunology, microbiology, genetics and protein and nucleic acid chemistry described herein are those well known and commonly used in the art. The methods and techniques of the present invention are generally performed according to conventional methods well known in the art and as described in various general and more specific references that are cited and discussed throughout the present specification unless otherwise indicated. See, e.g., Sambrook et al., Molecular Cloning: A Laboratory Manual, 2nd ed., Cold Spring Harbor Laboratory Press, Cold Spring Harbor, N.Y. (1989) and Ausubel et al., Current Protocols in Molecular Biology, Greene Publishing Associates (1992), and Harlow and Lane Antibodies: A Laboratory Manual, Cold Spring Harbor Laboratory Press, Cold Spring Harbor, N.Y. (1990). "MIF" or "macrophage migration inhibitory factor" refers to the protein, which is known as a critical mediator in the immune and inflammatory response, and as a counterregulator of glucocorticoids. MIF includes mammalian MIF, specifically human MIF (Swiss-Prot primary accession number: P14174), wherein the monomeric form is encoded as a 115 amino acid protein but is produced as a 114 amino acid protein due to cleavage of the initial methionine. "MIF" also includes "GIF" (glycosylation-inhibiting factor) and other forms of MIF such as fusion proteins of MIF. The numbering of the amino acids of MIF starts with the N-terminal methionine (amino acid 1) and ends with the C-terminal alanine (amino acid 115).

"oxidized MIF" or oxMIF is defined for the purposes of the invention as an isoform of MIF that occurs by treatment of MIF with mild oxidizing reagents, such as Cystine. As has been shown by the present inventors in earlier works, recombinant oxMIF that has been treated this way comprises isoform(s) of MIF that share structural rearrangements with oxMIF that (e.g.) occurs *in vivo* after challenge of animals with bacteria. redMIF is defined for the purposes of this invention as reduced MIF and is MIF which does not bind to RAB0, RAB9 and/or RAB4.

The anti-oxMIF antibodies described in this invention are able to discriminate between ox and red MIF, which are generated by mild oxidation or reduction, respectively, and are useful to specifically detect oxMIF. Discrimination between these conformers is assessed by ELISA or surface plasmon resonance, as is well known in the art.

**Assessing differential binding of the antibodies by Biacore.**

Binding kinetics of oxMIF and redMIF to antibody RAB9 and RAB0 was examined by surface plasmon resonance analysis using a Biacore 3000 System. The antibodies were coated on a CM5 (= carboxymethylated dextran)chip and recombinant MIF protein, pre-incubated with 0.2% Proclin300, were injected. (Proclin300 consists of oxidative isothiazolones that stabilize the oxMIF structure by avoiding a conversion of oxMIF to redMIF). In native HBS-EP buffer (= Biacore running buffer) without addition of ProClin300, none of the recombinant MIF proteins bound to RAB9, RAB0 or to the reference antibody (irrelevant isotype control antibody) used as negative (background) binding control.

In a preferred embodiment, oxMIF is MIF which is differentially bound by antibody RAB9, RAB4 and/or RAB0 or an antigen-binding fragment thereof, meaning that these antibodies do bind to oxMIF while redMIF is not bound by either one of these antibodies.

In other embodiments, the anti-oxMIF antibodies, e.g. the antibodies mentioned above or an antigen-binding portion thereof bind oxMIF with a $K_D$ of less than 100 nM, preferably a $K_D$ of less than 50 nM, even more preferred with a $K_D$ of less than 10 nM. Particularly preferred, the antibodies of this invention bind to oxMIF with a $K_D$ of less than 5 nM.

(Non-)binding of an antibody, e.g. RAB9, RAB4 or RAB0(to oxMIF or redMIF) can be determined as generally known to a person skilled in the art, examples being any one of the following methods: Differential Binding ELISA with recombinant MIF, or surface plasmon resonance using recombinant MIF in its reduced or oxidized state, like the well known Biacore assay, described above.

A preferred method for the determination of binding is surface plasmon resonance of an antibody to e.g. rec. (ox)MIF whereupon "binding" is meant to be represented by a $K_D$ of less than 100 nM preferably less than 50 nM, even more preferred less than 10 nM whereas the non-binding to redMIF is characterized by a $K_D$ of more than 400 nM. "Binding" and "specific binding" is used interchangeably here to denote the above. "Differential binding" in the context of this application means that a compound, in particular the antibodies as described herein, bind to oxMIF (e.g. with the $K_D$ values mentioned above) while they do not bind to redMIF (with non-binding again being defined as above).

An "antibody" refers to an intact antibody or an antigen-binding portion that competes with the intact antibody for (specific) binding. See generally, Fundamental Immunology, Ch. 7 (Paul, W., ed., 2nd ed. Raven Press, N.Y. (1989)).

The term antibody includes human antibodies, mammalian antibodies, isolated antibodies and genetically engineered forms such as chimeric, camelized or humanized antibodies, though not being limited thereto.

The term "antigen-binding portion" of an antibody refers to one or more fragments of an antibody that retain the ability to specifically bind to an antigen (e.g. (ox)MIF). Antigen-binding portions may be produced by recombinant DNA techniques or by enzymatic or chemical cleavage of intact antibodies. Antigen-binding portions include e.g. - though not limited thereto - the following: Fab, Fab', F(ab')2, Fv, and complementarity determining region (CDR) fragments, single-chain antibodies (scFv), chimeric antibodies, antibodies and polypeptides that contain at least a portion of an antibody that is sufficient to confer specific antigen binding to the polypeptide, i.e. ox or redMIF. From N-terminus to C-terminus, both the mature light and heavy chain variable domains comprise the regions FR1, CDR1, FR2, CDR2, FR3, CDR3 and FR4. The assignment of amino acids to each domain is in accordance with the definitions of Kabat, Sequences of Proteins of Immunological Interest (National Institutes of Health, Bethesda, Md. (1987 and 1991)), Chothia et al. J. Mol. Biol.196:901-917 (1987), or Chothia et al., Nature 342:878-883 (1989). An antibody or antigen-binding portion thereof can be derivatized or linked to another functional molecule (e.g., another peptide or protein). For example, an antibody or antigen- binding portion thereof can be functionally linked to one or more other molecular entities, such as another antibody (e.g., a bispecific antibody or a diabody), a detectable agent, a cytotoxic agent, a pharmaceutical agent, and/or a linking molecule.

The term "$K_D$" refers here, in accordance with the general knowledge of a person skilled in the art to the equilibrium dissociation constant of a particular antibody with the respective antigen. This equilibrium dissociation constant measures the propensity of a larger object (here: complex ox or red MIF/antibody) to separate, i.e. dissociate into smaller components (here: ox or redMIF and antibody).

The term "human antibody" refers to any antibody in which the variable and constant domains are human sequences. The term encompasses antibodies with sequences derived from human genes, but which have been changed, e.g. to decrease possible immunogenicity, increase affinity, eliminate cysteines that might cause undesirable folding, etc. The term encompasses such antibodies produced recombinantly in non-human cells, which might e.g. impart glycosylation not typical of human cells.

The term "humanized antibody" refers to antibodies comprising human sequences and containing also non-human sequences.

The term "camelized antibody" refers to antibodies wherein the antibody structure or sequences has been changed to more closely resemble antibodies from camels, also designated camelid antibodies. Methods for the design and production of camelized antibodies are part of the general knowledge of a person skilled in the art.

The term "chimeric antibody" refers to an antibody that comprises regions from two or more different species. The term "isolated antibody" or "isolated antigen-binding portion thereof" refers to an antibody or an antigen-binding portion thereof that has been identified and selected from an antibody source such as a phage display library or a B-cell repertoire.

The production of the anti-(ox)MIF antibodies according to the present invention includes any method for the generation of recombinant DNA by genetic engineering, e.g. via reverse transcription of RNA and/or amplification of DNA and cloning into expression vectors. In some embodiments, the vector is a viral vector, wherein additional DNA segments may be ligated into the viral genome. In some embodiments, the vector is capable of autonomous replication in a host cell into which it is introduced (e.g. bacterial vectors having a bacterial origin of replication and episomal mammalian vectors). In other embodiments, the vector (e.g. non-episomal mammalian vectors) can be integrated into the genome of a host cell upon introduction into the host cell, and thereby replicated along with the host genome. Moreover, certain vectors are capable of directing the expression of genes to which they are operatively linked. Such vectors are referred to herein as "recombinant expression vectors" (or simply, "expression vectors").

Anti-(ox)MIF antibodies can be produced *inter alia* by means of conventional expression vectors, such as bacterial vectors (e.g., pBR322 and its derivatives), or eukaryotic vectors. Those sequences that encode the antibody can be provided with regulatory sequences that regulate the replication, expression and/or secretion from the host cell. These regulatory sequences comprise, for instance, promoters (e.g., CMV or SV40) and signal sequences. The expression vectors can also comprise selection and amplification markers, such as the dihydrofolate reductase gene (DHFR), hygromycin-B-phosphotransferase, and thymidine-kinase. The components of the vectors used, such as selection markers, replicons, enhancers, can either be commercially obtained or prepared by means of conventional methods. The vectors can be constructed for the expression in various cell cultures, e.g., in mammalian cells such as CHO, COS, HEK293, NSO, fibroblasts, insect cells, yeast or bacteria such as *E.coli.* In some instances, cells are used that allow for optimal glycosylation of the expressed protein.

The anti-(ox)MIF antibody light chain gene(s) and the anti-(ox)MIF antibody heavy chain gene(s) can be inserted into separate vectors or the genes are inserted into the same expression vector. The antibody genes are inserted into the expression vector by standard methods, e.g., ligation of complementary restriction sites on the antibody gene fragment and vector, or blunt end ligation if no restriction sites are present.

The production of anti-(ox)MIF antibodies or antigen-binding fragments thereof may include any method known in the art for the introduction of recombinant DNA into eukaryotic cells by transfection, e.g. via electroporation or microinjection. For example, the recombinant expression of anti-(ox)MIF antibody can be achieved by introducing an expression plasmid containing the anti-(ox)MIF antibody encoding DNA sequence under the control of one or more regulating sequences such as a strong promoter, into a suitable host cell line, by an appropriate transfection method resulting in cells having the introduced sequences stably integrated into the genome. The lipofection method is an example of a transfection method which may be used according to the present invention.

The production of anti-(ox)MIF antibodies may also include any method known in the art for the cultivation of said transformed cells, e.g. in a continuous or batchwise manner, and the expression of the anti-(ox)MIF antibody, e.g. constitutive or upon induction. It is referred in particular to WO 2009/086920 for further reference for the production of anti-(ox)MIF antibodies. In a preferred embodiment, the anti-(ox)MIF antibodies as produced according to the present invention bind to oxMIF or an epitope thereof. Particularly preferred antibodies in accordance with the present invention are antibodies RAB9, RAB4 and/or RAB0 as well as RAM9, RAM4 and/or RAM0.

The sequences of these antibodies are partly also disclosed in WO 2009/086920; see in addition the sequence list of the present application and the following:

SEQ ID NO: 1 for the amino acid sequence of the light chain of RAB9:

```
DIQMTQSPSS  LSASVGDRVT  ITCRSSQRIM  TYLNWYQQKP  GKAPKLLIFV  ASHSQSGVPS

RFRGSGSETD  FTLTISGLQP  EDSATYYCQQ  SFWTPLTFGG  GTKVEIKRTV  AAPSVFIFPP

SDEQLKSGTA  SVVCLLNNFY  PREAKVQWKV  DNALQSGNSQ  ESVTEQDSKD  STYSLSSTLT

LSKADYEKHK  VYACEVTHQG  LSSPVTKSFN  RGEC,
```

SEQ ID NO: 2 for the amino acid sequence of the light chain of RAB4:

```
DIQMTQSPGT LSLSPGERAT LSCRASQGVS SSSLAWYQQK PGQAPRLLIY GTSSRATGIP
DRFSGSASGT DFTLTISRLQ PEDFAVYYCQ QYGRSLTFGG GTKVEIKRTV AAPSVFIFPP
SDEQLKSGTA SVVCLLNNFY PREAKVQWKV DNALQSGNSQ ESVTEQDSKD STYSLSSTLT
LSKADYEKHK VYACEVTHQG LSSPVTKSFN RGEC,
```

SEQ ID NO: 3 for the amino acid sequence of the light chain of RAB0:

```
DIQMTQSPGT LSLSPGERAT LSCRASQGVS SSSLAWYQQK PGQAPRLLIY GTSSRATGIP
DRFSGSASGT DFTLTISRLQ PEDFAVYYCQ QYGRSLTFGG GTKVEIKRTV AAPSVFIFPP
SDEQLKSGTA SVVCLLNNFY PREAKVQWKV DNALQSGNSQ ESVTEQDSKD STYSLSSTLT
LSKADYEKHK VYACEVTHQG LSSPVTKSFN RGEC,
```

SEQ ID NO: 4 for the amino acid sequence of the light chain of RAB2:

```
DIQMTQSPVT LSLSPGERAT LSCRASQSVR SSYLAWYQQK PGQTPRLLIY GASNRATGIP
DRFSGSGSGT DFTLTISRLE PEDFAVYYCQ QYGNSLTFGG GTKVEIK RTV AAPSVFIFPP
SDEQLKSGTA SVVCLLNNFY PREAKVQWKV DNALQSGNSQ ESVTEQDSKD STYSLSSTLT
LSKADYEKHK VYACEVTHQG LSSPVTKSFN RGEC,
```

SEQ ID NO: 5 for the amino acid sequence of the heavy chain of RAB9:

```
EVQLLESGGG LVQPGGSLRL SCAASGFTFS IYSMNWVRQA PGKGLEWVSS
IGSSGGTTYY ADSVKGRFTI SRDNSKNTLY LQMNSLRAED TAVYYCAGSQ
WLYGMDVWGQ GTTVTVSSAS TKGPSVFPLA PCSRSTSEST AALGCLVKDY
FPEPVTVSWN SGALTSGVHT FPAVLQSSGL YSLSSVVTVP SSSLGTKTYT
CNVDHKPSNT KVDKRVESKY GPPCPPCPAP EFLGGPSVFL FPPKPKDTLM
ISRTPEVTCV VVDVSQEDPE VQFNWYVDGV EVHNAKTKPR EEQFNSTYRV
VSVLTVLHQD WLNGKEYKCK VSNKGLPSSI EKTISKAKGQ PREPQVYTLP
PSQEEMTKNQ VSLTCLVKGF YPSDIAVEWE SNGQPENNYK TTPPVLDSDG
SFFLYSRLTV DKSRWQEGNV FSCSVMHEAL HNHYTQKSLS LSLGK,
```

SEQ ID NO: 6 for the amino acid sequence of the heavy chain of RAB4:

```
EVQLLESGGG LVQPGGSLRL SCAASGFTFS IYAMDWVRQA PGKGLEWVSG
IVPSGGFTKY ADSVKGRFTI SRDNSKNTLY LQMNSLRAED TAVYYCARVN
VIAVAGTGYY YYGMDVWGQG TTVTVSSAST KGPSVFPLAP CSRSTSESTA
ALGCLVKDYF PEPVTVSWNS GALTSGVHTF PAVLQSSGLY SLSSVVTVPS
SSLGTKTYTC NVDHKPSNTK VDKRVESKYG PPCPPCPAPE FLGGPSVFLF
PPKPKDTLMI SRTPEVTCVV VDVSQEDPEV QFNWYVDGVE VHNAKTKPRE
EQFNSTYRVV SVLTVLHQDW LNGKEYKCKV SNKGLPSSIE KTISKAKGQP
REPQVYTLPP SQEEMTKNQV SLTCLVKGFY PSDIAVEWES NGQPENNYKT
TPPVLDSDGS FFLYSRLTVD KSRWQEGNVF SCSVMHEALH NHYTQKSLSL
SLGK,
```

SEQ ID NO: 7 for the amino acid sequence of the heavy chain of RAB0:

```
EVQLLESGGG LVQPGGSLRL SCAASGFTFS WYAMDWVRQA PGKGLEWVSG
IYPSGGRTKY ADSVKGRFTI SRDNSKNTLY LQMNSLRAED TAVYYCARVN
VIAVAGTGYY YYGMDVWGQG TTVTVSSAST KGPSVFPLAP CSRSTSESTA
ALGCLVKDYF PEPVTVSWNS GALTSGVHTF PAVLQSSGLY SLSSVVTVPS
SSLGTKTYTC NVDHKPSNTK VDKRVESKYG PPCPPCPAPE FLGGPSVFLF
PPKPKDTLMI SRTPEVTCVV VDVSQEDPEV QFNWYVDGVE VHNAKTKPRE
EQFNSTYRVV SVLTVLHQDW LNGKEYKCKV SNKGLPSSIE KTISKAKGQP
REPQVYTLPP SQEEMTKNQV SLTCLVKGFY PSDIAVEWES NGQPENNYKT
TPPVLDSDGS FFLYSRLTVD KSRWQEGNVF SCSVMHEALH NHYTQKSLSL
SLGK,
```

SEQ ID NO: 8 for the amino acid sequence of the heavy chain of RAB2:

```
EVQLLESGGG LVQPGGSLRL SCAASGFTFS IYAMDWVRQA PGKGLEWVSG IVPSGGFTKY
ADSVKGRFTI SRDNSKNTLY LQMNSLRAED TAVYYCARVN VIAVAGTGYY YYGMDVWGQG
TTVTVSSAST KGPSVFPLAP CSRSTSESTA
ALGCLVKDYF PEPVTVSWNS GALTSGVHTF PAVLQSSGLY SLSSVVTVPS
SSLGTKTYTC NVDHKPSNTK VDKRVESKYG PPCPPCPAPE FLGGPSVFLF
PPKPKDTLMI SRTPEVTCVV VDVSQEDPEV QFNWYVDGVE VHNAKTKPRE
EQFNSTYRVV SVLTVLHQDW LNGKEYKCKV SNKGLPSSIE KTISKAKGQP
REPQVYTLPP SQEEMTKNQV SLTCLVKGFY PSDIAVEWES NGQPENNYKT
TPPVLDSDGS FFLYSRLTVD KSRWQEGNVF SCSVMHEALH NHYTQKSLSL
SLGK,
```

SEQ ID NO: 9 for the amino acid sequence of RAM0hc:

EVQLLESGGG LVQPGGSLRL SCAASGFTFS WYAMDWVRQA PGKGLEWVSG IYPSGGRTKY

ADSVKGRFTI SRDNSKNTLY LQMNSLRAED TAVYYCARVN VIAVAGTGYY YYGMDVWGQG

TTVTVSSAST KGPSVFPLAP SSKSTSGGTA ALGCLVKDYF PEPVTVSWNS GALTSGVHTF

PAVLQSSGLY SLSSVVTVPS

SSLGTQTYIC NVNHKPSNTK VDKRVEPKSC DKTHTCPPCP APELLGGPSV FLFPPKPKDT

LMISRTPEVT CVVVDVSHED PEVKFNWYVD GVEVHNAKTK PREEQYNSTY RVVSVLTVLH

QDWLNGKEYK CKVSNKALPA PIEKTISKAK GQPREPQVYT LPPSREEMTK NQVSLTCLVK

GFYPSDIAVE WESNGQPENN

YKTTPPVLDS DGSFFLYSKL TVDKSRWQQG NVFSCSVMHE ALHNHYTQKS LSLSPGK,

SEQ ID NO: 10 for the amino acid sequence of RAM0lc:

DIQMTQSPGT LSLSPGERAT LSCRASQGVS SSSLAWYQQK PGQAPRLLIY GTSSRATGIP

DRFSGSASGT DFTLTISRLQ PEDFAVYYCQ QYGRSLTFGG GTKVEIKRTV AAPSVFIFPP

SDEQLKSGTA SVVCLLNNFY PREAKVQWKV DNALQSGNSQ ESVTEQDSKD STYSLSSTLT

LSKADYEKHK VYACEVTHQG LSSPVTKSFN RGEC,

SEQ ID NO: 11 for the amino acid sequence of RAM9hc:

EVQLLESGGG LVQPGGSLRL SCAASGFTFS IYSMNWVRQA PGKGLEWVSS IGSSGGTTYY

ADSVKGRFTI SRDNSKNTLY LQMNSLRAED TAVYYCAGSQ WLYGMDVWGQ GTTVTVSSAS

TKGPSVFPLA PSSKSTSGGT AALGCLVKDY FPEPVTVSWN SGALTSGVHT FPAVLQSSGL

YSLSSVVTVP SSSLGTQTYI

CNVNHKPSNT KVDKRVEPKS CDKTHTCPPC PAPELLGGPS VFLFPPKPKD TLMISRTPEV

TCVVVDVSHE DPEVKFNWYV DGVEVHNAKT KPREEQYNST YRVVSVLTVL HQDWLNGKEY

KCKVSNKALP APIEKTISKA KGQPREPQVY TLPPSREEMT KNQVSLTCLV KGFYPSDIAV

EWESNGQPEN NYKTTPPVLD SDGSFFLYSK LTVDKSRWQQ GNVFSCSVMH EALHNHYTQK

SLSLSPGK,

SEQ ID NO: 12 for the amino acid sequence of RAM9lc:

DIQMTQSPSS LSASVGDRVT ITCRSSQRIM TYLNWYQQKP GKAPKLLIFV ASHSQSGVPS

RFRGSGSETD FTLTISGLQP EDSATYYCQQ SFWTPLTFGG GTKVEIKRTV AAPSVFIFPP

SDEQLKSGTA SVVCLLNNFY PREAKVQWKV DNALQSGNSQ ESVTEQDSKD STYSLSSTLT

LSKADYEKHK VYACEVTHQG LSSPVTKSFN RGEC,

SEQ ID NO: 13 for the amino acid sequence of RAM4hc:

```
EVQLLESGGG LVQPGGSLRL SCAASGFTFS IYAMDWVRQA PGKGLEWVSG IVPSGGFTKY
ADSVKGRFTI SRDNSKNTLY LQMNSLRAED TAVYYCARVN VIAVAGTGYY YYGMDVWGQG
TTVTVSSAST KGPSVFPLAP SSKSTSGGTA ALGCLVKDYF PEPVTVSWNS GALTSGVHTF
PAVLQSSGLY SLSSVVTVPS SSLGTQTYIC NVNHKPSNTK VDKRVEPKSC DKTHTCPPCP
APELLGGPSV FLFPPKPKDT LMISRTPEVT CVVVDVSHED PEVKFNWYVD GVEVHNAKTK
PREEQYNSTY RVVSVLTVLH QDWLNGKEYK CKVSNKALPA PIEKTISKAK GQPREPQVYT
LPPSREEMTK NQVSLTCLVK GFYPSDIAVE WESNGQPENN YKTTPPVLDS DGSFFLYSKL
TVDKSRWQQG NVFSCSVMHE ALHNHYTQKS LSLSPGK,


SEQ ID NO: 14 for the amino acid sequence of RAM4lc:
DIQMTQSPGT LSLSPGERAT LSCRASQGVS SSSLAWYQQK PGQAPRLLIY GTSSRATGIP
DRFSGSASGT DFTLTISRLQ PEDFAVYYCQ QYGRSLTFGG GTKVEIKRTV AAPSVFIFPP
SDEQLKSGTA SVVCLLNNFY PREAKVQWKV DNALQSGNSQ ESVTEQDSKD STYSLSSTLT
LSKADYEKHK VYACEVTHQG LSSPVTKSFN RGEC.
```

The anti-MIF antibody of the invention is preferably an isolated monoclonal antibody. The anti-MIF antibody can be an IgG, an IgM, an IgE, an IgA, or an IgD molecule. In other embodiments, the anti-MIF antibody is an IgG1, IgG2, IgG3 or IgG4 subclass. In other embodiments, the antibody is either subclass IgG1 or IgG4. In other embodiments, the antibody is subclass IgG4. In some embodiments, the IgG4 antibody has a single mutation changing the serine (serine228, according to the Kabat numbering scheme) to proline. Accordingly, the CPSC sub-sequence in the Fc region of IgG4 becomes CPPC, which is a sub-sequence in IgG1 (Angal et al. Mol Immunol. 1993, 30, 105-108).

Additionally, the production of anti-(ox)MIF antibodies may include any method known in the art for the purification of an antibody, e.g. via anion exchange chromatography or affinity chromatography. In one embodiment the anti-(ox)MIF antibody can be purified from cell culture supernatants by size exclusion chromatography.

The terms "center region" and "C-terminal region" of MIF refer to the region of human MIF comprising amino acids 35-68 and aa 86-115, respectively, preferably aa 50-68 and aa 86 to 102 of human MIF, respectively. Particularly preferred antibodies, which can be assayed by the present invention bind to either region aa 50-68 or region aa 86-102 of human MIF. This is also reflected by the binding of the preferred antibodies RAB0, RAB4 RAB2 and RAB9 as well as RAM4, RAM9 and RAM0 which bind as follows:

|  |  |
|---|---|
| RAB4 and RAM4: | aa 86-102 |
| RAB9 and RAM9: | aa 50-68 |
| RAB0 and RAM0: | aa 86-102 |
| RAB2: | aa 86-102 |

The term "epitope" includes any protein determinant capable of specific binding to an immunoglobulin or an antibody fragment. Epitopic determinants usually consist of chemically active surface groupings of molecules such as exposed amino acids, amino sugars, or other carbohydrate side chains and usually have specific three-dimensional structural characteristics, as well as specific charge characteristics.

The term "vector" refers to a nucleic acid molecule capable of transporting another nucleic acid to which it has been linked. In some embodiments, the vector is a plasmid, i.e., a circular double stranded DNA loop into which additional DNA segments may be ligated.

The term "host cell" refers to a cell line, which is capable to produce a recombinant protein after introducing an expression vector. The term "recombinant cell line", refers to a cell line into which a recombinant expression vector has been introduced. It should be understood that "recombinant cell line" means not only the particular subject cell line but also the progeny of such a cell line. Because certain modifications may occur in succeeding generations due to either mutation or environmental influences, such progeny may not, in fact, be identical to the parent cell, but are still included within the scope of the term "recombinant cell line" as used herein.

The host cell type according to the present invention is e.g. a COS cell, a CHO cell or e.g. an HEK293 cell, or any other host cell known to a person skilled in the art, thus also for example including bacterial cells, like e.g. *E.coli* cells. In one embodiment, the anti-MIF antibody is expressed in a DHFR-deficient CHO cell line, e.g., DXB11, and with the addition of G418 as a selection marker. When recombinant expression vectors encoding antibody genes are introduced into CHO host cells, the antibodies are produced by culturing the host cells for a period of time sufficient to allow for expression of the antibody in the host cells or secretion of the antibody into the culture medium in which the host cells are grown.

Anti-(ox)MIF antibodies can be recovered from the culture medium using standard protein purification methods.

Any anti-(ox)MIF antibody which is produced will have a given potency. If this antibody shall be formulated in a diagnostic or pharmaceutical formulation it is particularly important to ensure that this potency is known. Therefore, it is necessary to have an assay method which clearly and reliably measures and calculates this potency, e.g. as expressed in $IC_{50}$ values.

The preferred assay format of this invention is a modified Boyden chamber (Transwell®) assay, comprising two chambers, which allow cells to migrate and which allow counting the migrated cells preferably in the lower chamber after the assay has been finished. Both the Boyden chamber and Transwell® chamber assay are well known to a person skilled in the art. All well known devices which are used in the art for cell migration assays can be used in principle for the present assay method; the only pre-requisite being the provision of (at least) two chambers wherein the cells are provided in one chamber(e.g. the upper chamber) and wherein the antibodies are provided in the other chamber (e.g. the lower chamber). However, it is also possible to add the antibodies to the upper chamber, together with the cells. Apart from the Boyden chamber other well known assay formats can be used, e.g. a multiwell chamber, or a Dunn chamber (with concentric rings arranged on a slide. The chambers need to be inter-connected to allow diffusion and in particular migration of the cells in question. This is achieved by providing connecting membranes with respective pores. The pore size is selected as well known in the art depending on the cell type which is used in the assay. As examples which by no means are meant to be limiting, the following pore sizes are typically selected for an assay of the invention, e.g. a Boyden chamber assay:

| | |
|---|---|
| Astrocytes | 12 $\mu$m |
| Lymphocytes | 5 $\mu$m |
| Cancer cell lines | 8 $\mu$m |
| Macrophages | 5 $\mu$m |
| Endothelial cells | 8 $\mu$m |
| Monocytes | 5 $\mu$m |
| Epithelial cells | 8 $\mu$m |
| Neutrophils | 3 $\mu$m |
| Fibroblasts | 8 $\mu$m |
| Leukocytes | 3 $\mu$m |
| Slow moving cells | 12 $\mu$m. |

A preferred migration medium for use in the present invention is not particularly limited; Migration media are well known in the art In a preferred embodiment the medium is protein- and serum free.One example would be a serum-free RPMI-Medium: RPMI 1640 (Gibco, Cat.# 11835) with 10 mM Hepes, 1 mM Natrium Pyruvat, 4.5 g/L Glucose.

As an exemplary control, a medium with 10% FBS is used to induce cell migration (RPMI-Medium: RPMI 1640 (Gibco, Cat.# 11835) with 10% FBS inactivated, 10 mM Hepes, 1 mM Na-Pyruvat, 4.5 g/L Glucose, 0.05 mM $\beta$-Mercaptoethanol.

Preferred cells for the inventive assay are those cells, which are capable of migration. It is of particular importance to the present invention that the cells are cells which express (ox)MIF. The cells can express endogenous (ox)MIF or the (ox)MIF can be genetically engineered into these cells to be expressed by them. This in particular achieves the migration of the cells; that is to say, the cells stimulate their own migration by expressing (ox)MIF, preferably on their surface. It has been shown that these cells are particularly active in their migration and will be inhibited (i.e. slowed down) in this migration action if anti-(ox)MIF antibodies are coming into contact with the (ox)MIF of these cells. This contact will happen mostly on the division, e.g. membrane with pores, dividing the two chambers. If the antibodies in question have a high potency they will slow down the migratory action of the cells much more than in a case where the potency is low. The number of cells which have actually migrated through the division, e.g. the pores, from the upper to the lower chamber, is thus an indicator, how potent the antibodies are. A high number of cells migrated to the lower chamber equates a low potency (i.e. low inhibition of migration) while a low number of cells migrated to the lower chamber equates a high potency (i.e. high inhibition of migration).

Cells fulfilling these criteria are generally known to a person skilled in the art, however, in the present format, it is particularly preferred to use monocytic cells, e.g. (human) U937 cells (ATCC Cat.#: CRL-1593.2). If the potency of anti-oxMIF antibodies shall be determined the cells need to express (ox)MIF, e.g. on their cell surface, which can be determined e.g. by FACS (fluorescence activated cell sorting), as is well known to the person skilled in the art. They could recombinantly express MIF or endogenously express MIF, like e.g. cells taken from disease samples, e.g. cancer samples, as the present inventors have previously shown that oxMIF will only be expressed by cells during a disease state. The above preferred cells fulfil this criterion.

In a further preferred embodiment, the antibody is provided in a buffer system. The buffer system is preferably non-toxic and shows a good solubility for the antibody. More preferred, the buffer system comprises a moderately weak acid and its conjugate base, as well known to a person skilled in the art, e.g. PBS (phosphate buffered saline) or an N-substituted taurine buffer. A particularly preferred embodiment employs a glycine buffer (e.g. 100-350 mM glycine buffer, more preferred 200-300 mM, most preferred approximately 250 mM, at pH 4.5-5.5, preferably approximately pH 5.0).

The preferred temperature for the assay is at or around 37°C.

The present invention is further explained by the following examples which shall by no means be construed to limit the present scope of the invention which is defined by the claims enclosed herewith.

**EXAMPLES**

Example 1

Anti-MIF antibody RAM9 Chemokinesis assay; cell based assay
Intended Purpose:

The test was set up to test the functionality of Glycine-buffered anti-MIF RAM9 preparations (= test item) to inhibit random migration (= chemokinesis) of monocytic cells. It has been shown by the present inventors that this assay and respective method can be used as quality control test at the process step for the Final Drug Product (FDP).

**1) Rationale:**

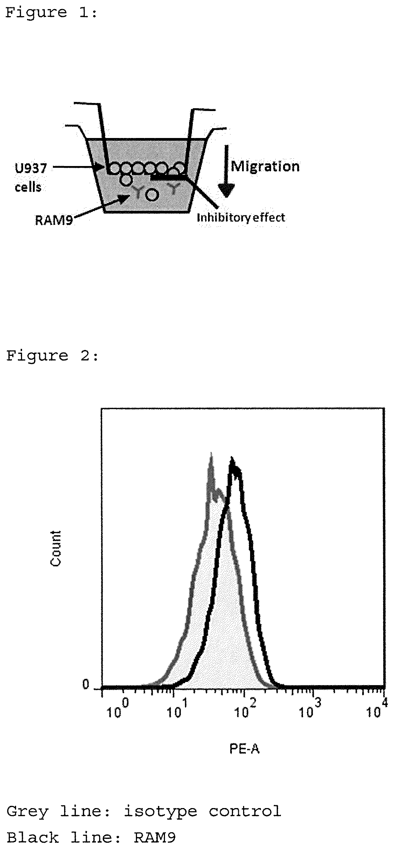

MIF is constitutively expressed in U397 (and other cancerous) monocytes and oxMIF is present on the cell surface of these cells (FACS data, see Figure 2) where it supports migratory functions. It is an important feature of the present invention to use cells which express (ox)MIF endogenously or exogenously, like cells from disease samples. This principle is based on the earlier finding of the present inventors that oxMIF is not present in healthy cells or tissues. The U397 cell line is a preferred example to carry out the present invention. It is an immortal cell line, not a primary cell line, from cancerous tissue.

Principle of testing method:

The capacity of anti-MIF antibodies to inhibit random migration (chemokinesis) of human monocytes is tested in a Transwell® assay (which is comparable to a Boyden chamber assay). The general set-up of the test method is shown in Figure 1. To that avail, serum starved monocytic cells (cell line: U937) were seeded into porous (5 $\mu$m) Transwell® inserts (i.e. "upper chamber") and cell migration towards different concentrations of anti-MIF antibody RAM9 (= test item in the lower chamber) was measured. The $IC_{50}$ was determined by a nonlinear regression equation (4-parameter logistic) of the number of migrated cells against the concentrations of RAM9 (logarithmic scale):

$$Y-(Ymax-Ymin)/(1+(X/IC_{50})Exp_{slope}+Ymin.$$

Details for testing method:

| Materials and equipment | |
|---|---|
| Transwell® Plates: | HTS 96 well-Transwell® Plates, 5 $\mu$m Pore Size Polycarbonate Membrane, Sterile, Polystyrene, Tissue Culture Treated (Corning, Cat.#: 3387) |
| Migration Medium: | Serum-free 0% RPMI-Medium: RPMI 1640 (Gibco, Cat.# 11835) with 10 mM Hepes, 1 mM Na-Pyruvat, PenStrep, 4.5 g/L Glucose |

(continued)

| 10% HG-full Medium: | 10% RPMI-Medium: RPMI 1640 (Gibco, Cat.# 11835) with 10% Fetal Bovine Serum (FBS) inactivated, 10 mM Hepes, 1 mM Na-Pyruvat, PenStrep, 4.5 g/L Glucose, 0.05 mM $\beta$-Mercaptoethanol (this medium is for us in regular cultivation, before the cells are put into serum-free medium and can be used as a positive control to induce cell migration by 10 % FBS in the inventive assay) |
|---|---|
| Sample Dilution Buffer: | 250 mM glycine-buffer, pH 5.0, sterile filtered |
| Cells: | U937-Cells with cell density of approx. $1 \times 10^6$ cells/ml, (www.atcc.org/ATCCAdvancedCatalogSearch/ProductDetails/tabid/452/Default. aspx?ATCCNum=CRL-1593.2&Template=cellBiology Cat.#: CRL-1593.2 (Total passage number from original ATCC vial: < 25) |
| Sample Dilution Plate: | sterile 96-U-Well-Plate e.g. Brand, Cat.#: 701316 |
| Equipment: | 37°C incubator, 5% $CO_2$, >80 % relative humidity (rH), e.g. Heraeus (CB_IN_17) CASY Cell Counting System, Innovatis (CB_SG_38) Cellavista™ system, Roche Centrifuge for 50 ml tubes; e.g. Heraeus Megafuge 1.0R (CB_ZF_05) 800 rpm equates 133 g (Rotor: #2704) |
| Antibodies: | RAM9, (GMP grade) |
| Control antibody (negativecontrol): | Synagis®, 100 mg/ml (Charge: 1006/1 0996) (isotype control antibody) |
| Positive control (p.c.): | 10 % HG-full medium (supra) without antibody, only with Antibody Dilution Buffer applied at the lower wells of the Transwell® Plate; Cells in Migration Medium are applied in the Transwell® inserts. |
| Buffer Control (b.c.): | Migration Medium without antibody, only Sample Dilution Buffer applied in the lower wells of the Transwell® Plate; Cells in Migration Medium are applied in the Transwell® inserts. |

## 2) Additional Information:

<u>Sample application:</u>

Each antibody dilution was applied six times (n=6). The mean value was used for further calculations. Minimal pipette volumes of 5 $\mu$l were used
<u>Controls:</u> Negative and Positive control on each plate
The assay has been performed in duplicate plates (two plates per lot test).

## 3) Layout (as shown in the diagram below):

Edge wells (of 96 well ELISA plate) are not to be used for RAM9 and control antibody samples to enhance reliability (only used for buffer control and positive control).

| b.c. | RAM9 30nM | RAM9 10nM | RAM9 3,33nM | RAM9 1,11nM | RAM9 0,37nM | RAM9 0,12nM | RAM9 0,04nM | Synagis 30nM | Synagis 10nM | Synagis 3,33nM | p.c. |
|---|---|---|---|---|---|---|---|---|---|---|---|
| b.c. | RAM9 30nM | RAM9 10nM | RAM9 3,33nM | RAM9 1,11nM | RAM9 0,37nM | RAM9 0,12nM | RAM9 0,04nM | Synagis 30nM | Synagis 10nM | Synagis 3,33nM | p.c. |
| b.c. | RAM9 30nM | RAM9 10nM | RAM9 3,33nM | RAM9 1,11nM | RAM9 0,37nM | RAM9 0,12nM | RAM9 0,04nM | Synagis 30nM | Synagis 10nM | Synagis 3,33nM | p.c. |
| b.c. | RAM9 30nM | RAM9 10nM | RAM9 3,33nM | RAM9 1,11nM | RAM9 0,37nM | RAM9 0,12nM | RAM9 0,04nM | Synagis 30nM | Synagis 10nM | Synagis 3,33nM | p.c. |

(continued)

| b.c. | RAM9 30nM | RAM9 10nM, | RAM9 3,33nM | RAM9 1,11nM | RAM9 0,37nM | RAM9 0,12nM | RAM9 0,04nM | Synagis 30nM | Synagis 10nM | Synagis 3,33nM | p.c. |
|------|-----------|------------|-------------|-------------|-------------|-------------|-------------|--------------|--------------|----------------|------|
| b.c. | RAM9 30nM | RAM9 10nM | RAM9 3,33nM | RAM9 1,11nM | RAM9 0,37nM | RAM9 0,12nM | RAM9 0,04nM | Synagis 30nM | Synagis 10nM | Synagis 3,33nM | p.c. |
| p.c. = positive control (serum) | | | | | | | | | | | |
| b.c. = background control (buffer) | | | | | | | | | | | |

**4) Procedure:**

Day 1:

a. the U937 cells were counted
b. a suitable amount of cell suspension was centrifuged at 800 rpm for 5 min at room temperature.
c. the supernatant was discarded and the cells were re-suspended in pre-warmed migration medium (wash).
d. A further centrifugation step with 800 rpm was carried out for 5 min at room temperature.
e. the supernatant was discarded.
f. Thereafter, the cells were resuspended in pre-warmed migration medium to 1 x $10^6$ cells/ml.
g. The cell suspension was incubated for 24 hours in the incubator.

Day 2:

a. The Transwell® plates were equilibrated as follows:

Apply 235 μl pre-warmed migration medium to all wells of the plate.
Put the Transwell® inserts in the plate and add 100 μl pre-warmed migration medium into the inserts. Incubate for at least 1 hour in the cell culture incubator.

b. The antibody-preparation was done as follows:

Final concentration of RAM9 in lower wells:

30 nM, 10 nM, 3,33 nM, 1,11 nM, 0,37 nM, 0,12 nM, 0,04 nM (1 μg/ml of IgG is calculated with 6,7 nM)

Final concentration of Synagis® in the lower wells:

30 nM, 10 nM, 3,33 nM (1 μg/ml of IgG is calculated with 6,7 nM)

The sample volumes were adjusted by adding the same volume of antibody dilution buffer. As it is highly recommended to do pre-dilution steps by use of dilution plates and multichannel pipettes so as to improve the mixing of the samples, pre-dilution steps were also carried out, as is known to a person skilled in the art.
c. The cell preparation was performed as follows:

The cells were counted and centrifuged at 800 rpm for 5 min at room temperature. The supernatant was discarded and the cells were washed once with pre-warmed migration medium. Another centriguation step was performed at 800 rpm for 5 min.

The supernatant was discarded and the pellet was resuspended to a cell count of 1 x $10^6$ cells/ml.

d. For the preparation of the plate the following steps were carried out:

The medium from the equilibration step was discarded (from the wells and the inserts).

Touching the insert's membranes was avoided and the inserts were placed in the Laminar Flow hood with the bottom side up! (Avoid air drying of the membranes)

e. 220 μl pre-warmed migration medium (or 10% HG-full medium = positive control) was added. 235 μl migration medium was added to unused wells.

The addition of antibodies was done as follows:

15 μl of the pre-diluted antibodies were applied with the multi-channel pipette to the wells of the Transwell® plate. Air bubbles in the wells were avoided!

f. The addition of the cells was performed as follows:

The Inserts were carefully attached to the Transwell® plate and 100 μl of the prepared cell suspension were added (1 x $10^6$ cells/ml) with a multi-channel pipette to every insert.

$\rightarrow$ Final cell numbers in the inserts: 1 x $10^5$ cells/well.

The Insert's membrane was not be touched with the pipette tips.

g. The plates were incubated over night(approx. 16 hours) in the cell culture incubator. <u>Day 3:</u>

a. The Inserts were discarded; the Transwell® plate was used for cell counts.
b. The cells were separated by pipetting up and down several times with the multi-channel pipette.
c. Air bubbles were removed.
d. The cells were allowed to sink down in the wells for at least 30 min before measuring.
e. The cells were counted by use of the Cellavista system (parameter settings **THP-1 AK** with operator settings for **Cell Confluence**).

The Evaluation (calculation of IC$_{50}$-values) is done as well known to a person skilled in the art, e.g. by use of a non-linear regression model with a 4-parameter fit and the following equation:

$$Y= (Ymax-Ymin)/(1+(X/IC_{50})Exp_{slope}+ Ymin$$

Exemplary range of acceptable IC$_{50}$-values for RAM9: $\geq$ 0.1 nM and $\leq$ 4 nM "Acceptable" in that regard shall mean if the calculated IC$_{50}$ of each plate is not within this range, the test has to be repeated. If the IC$_{50}$ of the repeated test is not within this range, the RAM9 antibody did not pass the test.

<u>Notice:</u>

For the calculation of the IC$_{50}$ of RAM9 e.g. at least five sequential concentrations (and fit values) should be included in the curve fit. (In the shown example, six concentrations (0,04 nM -10 nM antibody) have been used for calculation).

| Accuracy: | For this method, no national or international reference material is available. Therefore, an in-house reference (anti-MIF working standard Bulk drug substance (lot ORMFUFD09003 REF); 17.44 mg/ml) was used for determination of the IC$_{50}$ curves. Consistency of the assay was confirmed by use of this reference compound in every test. From the listed 25 experiments (=38 plates) a mean IC$_{50}$ of 0.8 was calculated. |
|---|---|
| | |

(continued)

| | | |
|---|---|---|
| **Precision:** | In order to determine the $IC_{50}$ of the reference compound described above, the Chemokinesis assay has been repeated 38 times (n=6 wells per concentration and assay) by 2 operators over a period of $\sim$ 7 months in the above example. The standard deviation from the 25 listed experiments (38 plates) is 0.7 $IC_{50}$: 0.8 $\pm$ 0.7 nM). When duplicate plates (26 plates from 13 experiments) are used for evaluation, a mean $IC_{50}$ of 0.8 and a standard deviation of 0.5 can be calculated ($IC_{50}$: 0.8 $\pm$ 0.5 nM). | |
| **Specificity:** | Dose dependent migration inhibition of U937 cells by RAM9(lot ORMFUFD09003 REF) could be shown repeatedly in several experiments. As negative control, three concentrations of another fully human IgG drug substance (antibody Palivizumab, commercial name Synagis®) have been used in the assays. General parameters that have not been changed during the qualification of the preferred embodiment:<br>▪ RAM9 was diluted in Glycine buffer and minimal pipetting volumes of 5 $\mu$l have been used.<br>▪ 24h Serum starvation of U937 monocytes.<br>▪ Equilibration of Transwell® plates in migration medium.<br>▪ Pre-dilution of antibody in Glycine buffer in 96 well plates (equivolume mixtures)<br>▪ Preparation of 1 x 10$^6$ cells/ml in fresh migration medium<br>▪ Addition of migration medium and diluted antibodies into the lower chamber of the 96 well plates.<br>▪ Addition of cell suspension (suspension in migration medium) into upper chamber (Transwell® insert).<br>▪ Overnight incubation of the plates (16 h, cell culture incubator).<br>▪ Removal of Transwell® inserts and counting of cells in the lower chamber (read out = cell numbers).<br>▪ Calculation of $IC_{50}$ by use of the excel solver function (non-linear regression, 4-Parameter fit) | |
| **Range:** | $IC_{50}$-Range: $\geq$ 0.1 nM and $\leq$ 4 nM | |
| | | |
| **Robustness:** | According to a preferred embodiment of this invention, freeze-thaw cycles of the test items (Glycine buffered preparations of anti-MIF antibody RAM9) are avoided. After Lot-changes of cells, it is preferred that the migration assay is re-evaluated by use of an accepted anti-MIF working standard (e.g. RAM9 BDS (bulk drug substance) material). The number of migrated cells (n) in the buffer control wells should be in a preferred embodiment n > 60 and n < 3000. If the number of migrated cells is below or above these limits, the test should be repeated ($IC_{50}$ values should not be taken) Thereby, it can be avoided that the number of cells is too small to carry out a meaningful statistic analysis or that there is no sufficient cell-cell-communication, and it is avoided that the number is too big, which could result in practice in a too pronounced cell-cell-communication. | |
| | | |
| **Equipment Qualification:** | Testing was performed on the following devices:<br>37°C incubator (5%$CO_2$, >80%rH), Heraeus<br>CASY Cell Counting System, Innovatis<br>Cellavista, Innovatis<br>Centrifuge Heraeus Megafuge 1.0R<br>This equipment is evaluated as acceptable due to design of the test. | |
| 5) **Conclusion:** The method is qualified for its intended purpose, i.e. it can be successfully used for testing the potency of anti-(ox)MIF antibodies. In particular, it is very suitable for testing of clinical phase I + II material. | | |

**Example 2**

In principle, the same assay method was used to determine whether the antibodies could be provided together with the cells in the upper chamber.

Short summary:

Cell Migration Assay: HTS Transwell plates (Corning): $5\mu m$

* cells: U937 (10th passage), overnight starving

* RAM9 antibody: 30nM - 0,04 nM, pipetted into inserts

* Synagis® in Glycine: 30 nM; 10 nM; 3,3 nM pipetted into inserts

* Neg. control: Glycine; Pos. control: FBS (fetal bovine serum)

* Incubation: 2 plates, 16 h

* Cellavista® used for calculation of results

Results:

Plate 1

| Conc. Antibody (nM) | measured | fit |
|---|---|---|
| 0 | 1997,6 | |
| 0,04 | 2452,7 | 2498,3 |
| 0,12 | 2490,8 | 2388,4 |
| 0,37 | 2019,2 | 2106,0 |
| 1,1 | 1642,0 | 1621,2 |
| 3,3 | 1179,6 | 1115,5 |
| 10 | 688,0 | 810,8 |
| 30 | 753,7 | 685,7 |
| Max | =A | 2555 |
| Slope | =B | 1,0 |
| **IC 50** | **=C** | **1,2** |
| Min | =D | 620 |
| sumxmy2 | | 44358 |
| **Fit:** I%=(A-D)/(1+(X/C)ExpB + D (=sigmoid curve) | | |

Plate 2

| CKonc. Antibody (nM) | measured | fit |
|---|---|---|
| 0 | 2183,1 | |
| 0,04 | 2562,8 | 2646,5 |
| 0,12 | 2685,2 | 2575,9 |
| 0,37 | 2354,2 | 2357,3 |
| 1,1 | 1872,5 | 1939,5 |
| 3,3 | 1621,2 | 1527,4 |
| 10 | 1263,3 | 1323,0 |

(continued)

| CKonc. Antibody (nM) | measured | fit |
|---|---|---|
| 30 | 1266,7 | 1256,2 |
| Max | =A | 2674 |
| Slope | =B | 1,2 |
| **IC 50** | **=C** | **1,1** |
| Min | =D | 1230 |
| sumxmy2 | | 35893 |
| Fit: I%=(A-D)/(1+(X/C)ExpB + D (=sigmoid curve) | | |

Conclusion:

The assay method is again suitable for its intended purpose.

The present assay sets a new (industrial) standard with respect to assay robustness and precision that will allow to test MIF specific antibodies/drugs for their potency according to FDA bioassay guidelines; the actual assay is a qualified test that is sufficient to test anti-MIF final drug product lots until clinical Phase III. By application of monocytic cells from other species (e.g. from rats) the assay can also be used to support species comparability studies of MIF inhibiting antibodies/molecules without the need for the use of recombinant MIF.

The assay is easy to use and does not require the use of recombinant MIF protein. Based on the mechanism of action, the bioassay was accepted by the FDA for the assessment of anti-MIF antibody potency in the context of an inflammatory disease. Other assay principles/formats could be demanded by regulatory agencies in order to assess the *in vitro* potency of anti-MIF antibodies/drugs in other indications (e.g. cancer).

BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

Baxter Healthcare S.A.

Thurgauerstr. 130

8152 Glattpark (Opfikon), Switzerland

Baxter International Inc.

One Baxter Parkway

Deerfield, Illinois 60015, USA

RECEIPT IN THE CASE OF AN ORIGINAL DEPOSIT
issued pursuant to Rule 7.1 by the
INTERNATIONAL DEPOSITARY AUTHORITY
identified at the bottom of this page

---

**I. IDENTIFICATION OF THE MICROORGANISM**

| Identification reference given by the DEPOSITOR: <br> GA.643-22.pRAB4lc | Accession number given by the <br> INTERNATIONAL DEPOSITARY AUTHORITY: <br><br> DSM 25110 |
|---|---|

**II. SCIENTIFIC DESCRIPTION AND/OR PROPOSED TAXONOMIC DESIGNATION**

The microorganism identified under I. above was accompanied by:

( x ) a scientific description
( x ) a proposed taxonomic designation

(Mark with a cross where applicable).

**III. RECEIPT AND ACCEPTANCE**

This International Depositary Authority accepts the microorganism identified under I. above, which was received by it on 2011-08-31 (Date of the original deposit)[1].

**IV. RECEIPT OF REQUEST FOR CONVERSION**

The microorganism identified under I above was received by this International Depositary Authority on                              (date of original deposit)
and a request to convert the original deposit to a deposit under the Budapest Treaty was received by it on                              (date of receipt of request
for conversion).

**V. INTERNATIONAL DEPOSITARY AUTHORITY**

| Name: | DSMZ-DEUTSCHE SAMMLUNG VON <br> MIKROORGANISMEN UND ZELLKULTUREN GmbH | Signature(s) of person(s) having the power to represent the <br> International Depositary Authority or of authorized official(s): |
|---|---|---|
| Address: | Inhoffenstr. 7 B <br> D-38124 Braunschweig | Date: 2011-09-02 |

[1] Where Rule 6.4 (d) applies, such date is the date on which the status of international depositary authority was acquired.

Form DSMZ-BP/4 (sole page) 08/2006

# EP 2 929 348 B1

INTERNATIONAL FORM

Baxter Healthcare S.A.

Thurgauerstr. 130

8152 Glattpark (Opfikon), Switzerland

Baxter International Inc.

One Baxter Parkway

Deerfield, Illinois 60015, USA

RECEIPT IN THE CASE OF AN ORIGINAL DEPOSIT
issued pursuant to Rule 7.1 by the
INTERNATIONAL DEPOSITARY AUTHORITY
identified at the bottom of this page

---

**I. IDENTIFICATION OF THE MICROORGANISM**

| Identification reference given by the DEPOSITOR: | Accession number given by the INTERNATIONAL DEPOSITARY AUTHORITY: |
|---|---|
| GA.661-01.pRAB9lc | DSM 25111 |

**II. SCIENTIFIC DESCRIPTION AND/OR PROPOSED TAXONOMIC DESIGNATION**

The microorganism identified under I. above was accompanied by:

( x ) a scientific description
( x ) a proposed taxonomic designation

(Mark with a cross where applicable).

**III. RECEIPT AND ACCEPTANCE**

This International Depositary Authority accepts the microorganism identified under I. above, which was received by it on 2011-08-31 (Date of the original deposit)[1].

**IV. RECEIPT OF REQUEST FOR CONVERSION**

The microorganism identified under I above was received by this International Depositary Authority on (date of original deposit)
and a request to convert the original deposit to a deposit under the Budapest Treaty was received by it on (date of receipt of request for conversion).

**V. INTERNATIONAL DEPOSITARY AUTHORITY**

| Name: | DSMZ-DEUTSCHE SAMMLUNG VON MIKROORGANISMEN UND ZELLKULTUREN GmbH | Signature(s) of person(s) having the power to represent the International Depositary Authority or of authorized official(s): |
|---|---|---|
| Address: | Inhoffenstr. 7 B D-38124 Braunschweig | Date: 2011-09-02 |

[1] Where Rule 6.4 (d) applies, such date is the date on which the status of international depositary authority was acquired.

Form DSMZ-BP/4 (sole page) 08/2006

22

BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

Baxter Healthcare S.A.

Thurgauerstr. 130

8152 Glattpark (Opfikon), Switzerland

Baxter International Inc.

One Baxter Parkway

Deerfield, Illinois 60015, USA

RECEIPT IN THE CASE OF AN ORIGINAL DEPOSIT
issued pursuant to Rule 7.1 by the
INTERNATIONAL DEPOSITARY AUTHORITY
identified at the bottom of this page

| I. IDENTIFICATION OF THE MICROORGANISM | |
|---|---|
| Identification reference given by the DEPOSITOR:<br><br>GA.736-12.pRAB4G4hc | Accession number given by the<br>INTERNATIONAL DEPOSITARY AUTHORITY:<br><br>DSM 25112 |

II. SCIENTIFIC DESCRIPTION AND/OR PROPOSED TAXONOMIC DESIGNATION

The microorganism identified under I. above was accompanied by:

( X ) a scientific description
( X ) a proposed taxonomic designation

(Mark with a cross where applicable).

III. RECEIPT AND ACCEPTANCE

This International Depositary Authority accepts the microorganism identified under I. above, which was received by it on 2011-08-31
(Date of the original deposit)[1].

IV. RECEIPT OF REQUEST FOR CONVERSION

The microorganism identified under I above was received by this International Depositary Authority on          (date of original deposit)

and a request to convert the original deposit to a deposit under the Budapest Treaty was received by it on          (date of receipt of request
for conversion).

V. INTERNATIONAL DEPOSITARY AUTHORITY

| Name: | DSMZ-DEUTSCHE SAMMLUNG VON<br>MIKROORGANISMEN UND ZELLKULTUREN GmbH | Signature(s) of person(s) having the power to represent the<br>International Depositary Authority or of authorized official(s): |
|---|---|---|
| Address: | Inhoffenstr. 7 B<br>D-38124 Braunschweig | |
| | | Date: 2011-09-02 |

[1] Where Rule 6.4 (d) applies, such date is the date on which the status of international depositary authority was acquired.

Form DSMZ-BP/4 (sole page) 08/2006

# EP 2 929 348 B1

BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

Baxter Healthcare S.A.

Thurgauerstr. 130

8152 Glattpark (Opfikon), Switzerland

Baxter International Inc.

One Baxter Parkway

Deerfield, Illinois 60015, USA

RECEIPT IN THE CASE OF AN ORIGINAL DEPOSIT
issued pursuant to Rule 7.1 by the
INTERNATIONAL DEPOSITARY AUTHORITY
identified at the bottom of this page

---

**I. IDENTIFICATION OF THE MICROORGANISM**

| Identification reference given by the DEPOSITOR: | Accession number given by the |
|---|---|
| GA.757-03.pRAB9G4hc | INTERNATIONAL DEPOSITARY AUTHORITY: |
| | DSM 25113 |

**II. SCIENTIFIC DESCRIPTION AND/OR PROPOSED TAXONOMIC DESIGNATION**

The microorganism identified under I. above was accompanied by:

( x ) a scientific description
( x ) a proposed taxonomic designation

(Mark with a cross where applicable).

**III. RECEIPT AND ACCEPTANCE**

This International Depositary Authority accepts the microorganism identified under I. above, which was received by it on 2011-08-31 (Date of the original deposit)[1].

**IV. RECEIPT OF REQUEST FOR CONVERSION**

| The microorganism identified under I above was received by this International Depositary Authority on | (date of original deposit) |
| and a request to convert the original deposit to a deposit under the Budapest Treaty was received by it on for conversion). | (date of receipt of request |

**V. INTERNATIONAL DEPOSITARY AUTHORITY**

| Name: | DSMZ-DEUTSCHE SAMMLUNG VON MIKROORGANISMEN UND ZELLKULTUREN GmbH | Signature(s) of person(s) having the power to represent the International Depositary Authority or of authorized official(s): |
|---|---|---|
| Address: | Inhoffenstr. 7 B D-38124 Braunschweig | |
| | | Date: 2011-09-02 |

[1] Where Rule 6.4 (d) applies, such date is the date on which the status of international depositary authority was acquired.

Form DSMZ-BP/4 (sole page) 08/2006

24

# EP 2 929 348 B1

BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

Baxter Healthcare S.A.

Thurgauerstr. 130

8152 Glattpark (Opfikon), Switzerland

Baxter International Inc.

One Baxter Parkway

Deerfield, Illinois 60015, USA

RECEIPT IN THE CASE OF AN ORIGINAL DEPOSIT
issued pursuant to Rule 7.1 by the
INTERNATIONAL DEPOSITARY AUTHORITY
identified at the bottom of this page

---

**I. IDENTIFICATION OF THE MICROORGANISM**

| Identification reference given by the DEPOSITOR:<br>GA.782-06.pRAB01c | Accession number given by the<br>INTERNATIONAL DEPOSITARY AUTHORITY:<br><br>DSM 25114 |
|---|---|

**II. SCIENTIFIC DESCRIPTION AND/OR PROPOSED TAXONOMIC DESIGNATION**

The microorganism identified under I. above was accompanied by:

      ( x )  a scientific description
      ( x )  a proposed taxonomic designation

(Mark with a cross where applicable).

**III. RECEIPT AND ACCEPTANCE**

This International Depositary Authority accepts the microorganism identified under I. above, which was received by it on 2011-08-31 (Date of the original deposit)[1].

**IV. RECEIPT OF REQUEST FOR CONVERSION**

The microorganism identified under I above was received by this International Depositary Authority on         (date of original deposit)
and a request to convert the original deposit to a deposit under the Budapest Treaty was received by it on         (date of receipt of request for conversion).

**V. INTERNATIONAL DEPOSITARY AUTHORITY**

| Name: | DSMZ-DEUTSCHE SAMMLUNG VON<br>MIKROORGANISMEN UND ZELLKULTUREN GmbH | Signature(s) of person(s) having the power to represent the<br>International Depositary Authority or of authorized official(s): |
|---|---|---|
| Address: | Inhoffenstr. 7 B<br>D-38124 Braunschweig | |

Date: 2011-09-02

[1] Where Rule 6.4 (d) applies, such date is the date on which the status of international depositary authority was acquired.

Form DSMZ-BP/4 (sole page) 08/2006

BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

Baxter Healthcare S.A.

Thurgauerstr. 130

8152 Glattpark (Opfikon), Switzerland

Baxter International Inc.

One Baxter Parkway

Deerfield, Illinois 60015, USA

RECEIPT IN THE CASE OF AN ORIGINAL DEPOSIT
issued pursuant to Rule 7.1 by the
INTERNATIONAL DEPOSITARY AUTHORITY
identified at the bottom of this page

---

**I. IDENTIFICATION OF THE MICROORGANISM**

| Identification reference given by the DEPOSITOR:<br><br>GA.783-34.pRAB0G4hc | Accession number given by the<br>INTERNATIONAL DEPOSITARY AUTHORITY:<br><br>DSM 25115 |
| --- | --- |

**II. SCIENTIFIC DESCRIPTION AND/OR PROPOSED TAXONOMIC DESIGNATION**

The microorganism identified under I. above was accompanied by:

    ( x ) a scientific description
    ( x ) a proposed taxonomic designation

(Mark with a cross where applicable).

**III. RECEIPT AND ACCEPTANCE**

This International Depositary Authority accepts the microorganism identified under I. above, which was received by it on 2011-08-31 (Date of the original deposit)[1].

**IV. RECEIPT OF REQUEST FOR CONVERSION**

The microorganism identified under I above was received by this International Depositary Authority on      (date of original deposit)

and a request to convert the original deposit to a deposit under the Budapest Treaty was received by it on      (date of receipt of request for conversion).

**V. INTERNATIONAL DEPOSITARY AUTHORITY**

| Name: | DSMZ-DEUTSCHE SAMMLUNG VON MIKROORGANISMEN UND ZELLKULTUREN GmbH | Signature(s) of person(s) having the power to represent the International Depositary Authority or of authorized official(s): |
| --- | --- | --- |
| Address: | Inhoffenstr. 7 B<br>D-38124 Braunschweig | |
| | | Date: 2011-09-02 |

[1] Where Rule 6.4 (d) applies, such date is the date on which the status of international depositary authority was acquired.

Form DSMZ-BP/4 (sole page) 08/2006

# EP 2 929 348 B1

BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

Baxter Healthcare S.A.

Thurgauerstr. 130, 8152 Glattpark

(Opfikon), Switzerland

Baxter International Inc.

One Baxter Parkway

Deerfield, Illinois 60015, USA

RECEIPT IN THE CASE OF AN ORIGINAL DEPOSIT
issued pursuant to Rule 7.1 by the
INTERNATIONAL DEPOSITARY AUTHORITY
identified at the bottom of this page

---

**I. IDENTIFICATION OF THE MICROORGANISM**

| Identification reference given by the DEPOSITOR:<br><br>GA.661-01.pRAM9lc | Accession number given by the<br>INTERNATIONAL DEPOSITARY AUTHORITY:<br><br>DSM 25859 |
|---|---|

**II. SCIENTIFIC DESCRIPTION AND/OR PROPOSED TAXONOMIC DESIGNATION**

The microorganism identified under I. above was accompanied by:

      ( X ) a scientific description
      ( X ) a proposed taxonomic designation

(Mark with a cross where applicable).

**III. RECEIPT AND ACCEPTANCE**

This International Depositary Authority accepts the microorganism identified under I. above, which was received by it on 2012-04-12 (Date of the original deposit)[1].

**IV. RECEIPT OF REQUEST FOR CONVERSION**

The microorganism identified under I above was received by this International Depositary Authority on (date of original deposit)
and a request to convert the original deposit to a deposit under the Budapest Treaty was received by it on (date of receipt of request for conversion).

**V. INTERNATIONAL DEPOSITARY AUTHORITY**

| Name: Leibniz Institute DSMZ-German Collection of Microorganisms and Cell Cultures<br><br>Address: Inhoffenstr. 7 B<br>D-38124 Braunschweig | Signature(s) of person(s) having the power to represent the International Depositary Authority or of authorized official(s):<br><br>*V. Werk*<br><br>Date: 2012-04-23 |
|---|---|

[1] Where Rule 6.4 (d) applies, such date is the date on which the status of international depositary authority was acquired.

Form DSMZ-BP/4 (sole page) 02/2012

# EP 2 929 348 B1

BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

Baxter Healthcare S.A.

Thurgauerstr. 130, 8152 Glattpark

(Opfikon), Switzerland

Baxter International Inc.

One Baxter Parkway

Deerfield, Illinois 60015, USA

RECEIPT IN THE CASE OF AN ORIGINAL DEPOSIT
issued pursuant to Rule 7.1 by the
INTERNATIONAL DEPOSITARY AUTHORITY
identified at the bottom of this page

---

**I. IDENTIFICATION OF THE MICROORGANISM**

| Identification reference given by the DEPOSITOR: | Accession number given by the INTERNATIONAL DEPOSITARY AUTHORITY: |
|---|---|
| GA.662-01.pRAM9hc | DSM 25860 |

**II. SCIENTIFIC DESCRIPTION AND/OR PROPOSED TAXONOMIC DESIGNATION**

The microorganism identified under I. above was accompanied by:

( x ) a scientific description
( x ) a proposed taxonomic designation

(Mark with a cross where applicable).

**III. RECEIPT AND ACCEPTANCE**

This International Depositary Authority accepts the microorganism identified under I. above, which was received by it on 2012-04-12 (Date of the original deposit)[1].

**IV. RECEIPT OF REQUEST FOR CONVERSION**

The microorganism identified under I above was received by this International Depositary Authority on (date of original deposit) and a request to convert the original deposit to a deposit under the Budapest Treaty was received by it on (date of receipt of request for conversion).

**V. INTERNATIONAL DEPOSITARY AUTHORITY**

| Name: Leibniz Institute DSMZ-German Collection of Microorganisms and Cell Cultures | Signature(s) of person(s) having the power to represent the International Depositary Authority or of authorized official(s): |
|---|---|
| Address: Inhoffenstr. 7 B D-38124 Braunschweig | Date: 2012-04-23 |

[1] Where Rule 6.4 (d) applies, such date is the date on which the status of international depositary authority was acquired.

Form DSMZ-BP/4 (sole page) 02/2012

28

# EP 2 929 348 B1

BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

Baxter Healthcare S.A.

Thurgauerstr. 130, 8152 Glattpark

(Opfikon), Switzerland

Baxter International Inc.

One Baxter Parkway

Deerfield, Illinois 60015, USA

RECEIPT IN THE CASE OF AN ORIGINAL DEPOSIT
issued pursuant to Rule 7.1 by the
INTERNATIONAL DEPOSITARY AUTHORITY
identified at the bottom of this page

---

**I. IDENTIFICATION OF THE MICROORGANISM**

| Identification reference given by the DEPOSITOR:<br><br>GA.906-04.pRAM4lc | Accession number given by the<br>INTERNATIONAL DEPOSITARY AUTHORITY:<br><br>DSM 25861 |
|---|---|

**II. SCIENTIFIC DESCRIPTION AND/OR PROPOSED TAXONOMIC DESIGNATION**

The microorganism identified under I. above was accompanied by:

     ( X ) a scientific description
     ( X ) a proposed taxonomic designation

(Mark with a cross where applicable).

**III. RECEIPT AND ACCEPTANCE**

This International Depositary Authority accepts the microorganism identified under I. above, which was received by it on 2012-04-12 (Date of the original deposit)[1].

**IV. RECEIPT OF REQUEST FOR CONVERSION**

| The microorganism identified under I above was received by this International Depositary Authority on | (date of original deposit) |
|---|---|
| and a request to convert the original deposit to a deposit under the Budapest Treaty was received by it on for conversion). | (date of receipt of request |

**V. INTERNATIONAL DEPOSITARY AUTHORITY**

| Name: Leibniz Institute DSMZ-German Collection of<br>Microorganisms and Cell Cultures<br><br>Address: Inhoffenstr. 7 B<br>D-38124 Braunschweig | Signature(s) of person(s) having the power to represent the<br>International Depositary Authority or of authorized official(s):<br><br>*V. Werks*<br><br>Date: 2012-04-23 |
|---|---|

[1] Where Rule 6.4 (d) applies, such date is the date on which the status of international depositary authority was acquired.

Form DSMZ-BP/4 (sole page) 02/2012

BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

Baxter Healthcare S.A.

Thurgauerstr. 130, 8152 Glattpark

(Opfikon), Switzerland

Baxter International Inc.

One Baxter Parkway

Deerfield, Illinois 60015, USA

RECEIPT IN THE CASE OF AN ORIGINAL DEPOSIT
issued pursuant to Rule 7.1 by the
INTERNATIONAL DEPOSITARY AUTHORITY
identified at the bottom of this page

---

**I. IDENTIFICATION OF THE MICROORGANISM**

| Identification reference given by the DEPOSITOR:<br>GA.657-02.pRAM4hc | Accession number given by the<br>INTERNATIONAL DEPOSITARY AUTHORITY:<br><br>DSM 25862 |
|---|---|

**II. SCIENTIFIC DESCRIPTION AND/OR PROPOSED TAXONOMIC DESIGNATION**

The microorganism identified under I. above was accompanied by:

( x )  a scientific description
( x )  a proposed taxonomic designation

(Mark with a cross where applicable).

**III. RECEIPT AND ACCEPTANCE**

This International Depositary Authority accepts the microorganism identified under I. above, which was received by it on  2012-04-12
(Date of the original deposit)[1].

**IV. RECEIPT OF REQUEST FOR CONVERSION**

The microorganism identified under I above was received by this International Depositary Authority on  (date of original deposit)

and a request to convert the original deposit to a deposit under the Budapest Treaty was received by it on  (date of receipt of request
for conversion).

**V. INTERNATIONAL DEPOSITARY AUTHORITY**

| Name: Leibniz Institute DSMZ–German Collection of<br>Microorganisms and Cell Cultures<br><br>Address: Inhoffenstr. 7 B<br>D-38124 Braunschweig | Signature(s) of person(s) having the power to represent the<br>International Depositary Authority or of authorized official(s):<br><br><br>Date: 2012-04-23 |
|---|---|

---

[1]  Where Rule 6.4 (d) applies, such date is the date on which the status of international depositary authority was acquired.

Form DSMZ-BP/4 (sole page) 02/2012

BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

Baxter Healthcare S.A.

Thurgauerstr. 130, 8152 Glattpark

(Opfikon), Switzerland

Baxter International Inc.

One Baxter Parkway

Deerfield, Illinois 60015, USA

RECEIPT IN THE CASE OF AN ORIGINAL DEPOSIT
issued pursuant to Rule 7.1 by the
INTERNATIONAL DEPOSITARY AUTHORITY
identified at the bottom of this page

| I. IDENTIFICATION OF THE MICROORGANISM | |
|---|---|
| Identification reference given by the DEPOSITOR: <br><br> GA.906-01.pRAM01c | Accession number given by the <br> INTERNATIONAL DEPOSITARY AUTHORITY: <br><br> DSM 25863 |

**II. SCIENTIFIC DESCRIPTION AND/OR PROPOSED TAXONOMIC DESIGNATION**

The microorganism identified under I. above was accompanied by:

( X ) a scientific description
( X ) a proposed taxonomic designation

(Mark with a cross where applicable).

**III. RECEIPT AND ACCEPTANCE**

This International Depositary Authority accepts the microorganism identified under I. above, which was received by it on 2012-04-12
(Date of the original deposit)[1].

**IV. RECEIPT OF REQUEST FOR CONVERSION**

The microorganism identified under I above was received by this International Depositary Authority on     (date of original deposit)

and a request to convert the original deposit to a deposit under the Budapest Treaty was received by it on     (date of receipt of request
for conversion).

**V. INTERNATIONAL DEPOSITARY AUTHORITY**

| | |
|---|---|
| Name:    Leibniz Institute DSMZ-German Collection of <br>           Microorganisms and Cell Cultures <br><br> Address:   Inhoffenstr. 7 B <br>            D-38124 Braunschweig | Signature(s) of person(s) having the power to represent the <br> International Depositary Authority or of authorized official(s): <br><br><br> Date: 2012-04-23 |

[1] Where Rule 6.4 (d) applies, such date is the date on which the status of international depositary authority was acquired.

Form DSMZ-BP/4 (sole page) 02/2012

# EP 2 929 348 B1

INTERNATIONAL FORM

Baxter Healthcare S.A.

Thurgauerstr. 130, 8152 Glattpark

(Opfikon), Switzerland

Baxter International Inc.

One Baxter Parkway

Deerfield, Illinois 60015, USA

RECEIPT IN THE CASE OF AN ORIGINAL DEPOSIT
issued pursuant to Rule 7.1 by the
INTERNATIONAL DEPOSITARY AUTHORITY
identified at the bottom of this page

---

**I. IDENTIFICATION OF THE MICROORGANISM**

| Identification reference given by the DEPOSITOR:<br><br>GA.784-01.pRAM0hc | Accession number given by the<br>INTERNATIONAL DEPOSITARY AUTHORITY:<br><br>DSM  25864 |
|---|---|

**II. SCIENTIFIC DESCRIPTION AND/OR PROPOSED TAXONOMIC DESIGNATION**

The microorganism identified under I. above was accompanied by:

( x )  a scientific description
( x )  a proposed taxonomic designation

(Mark with a cross where applicable).

**III. RECEIPT AND ACCEPTANCE**

This International Depositary Authority accepts the microorganism identified under I. above, which was received by it on 2012-04-12 (Date of the original deposit)[1].

**IV. RECEIPT OF REQUEST FOR CONVERSION**

| The microorganism identified under 1 above was received by this International Depositary Authority on | (date of original deposit) |
| and a request to convert the original deposit to a deposit under the Budapest Treaty was received by it on for conversion). | (date of receipt of request |

**V. INTERNATIONAL DEPOSITARY AUTHORITY**

| Name:  Leibniz Institute DSMZ-German Collection of<br>Microorganisms and Cell Cultures<br><br>Address:  Inhoffenstr. 7 B<br>D-38124 Braunschweig | Signature(s) of person(s) having the power to represent the<br>International Depositary Authority or of authorized official(s):<br><br><br>Date: 2012-04-23 |
|---|---|

[1] Where Rule 6.4 (d) applies, such date is the date on which the status of international depositary authority was acquired.

Form DSMZ-BP/4 (sole page) 02/2012

**Claims**

1. Assay method for determining the potency of anti-MIF antibodies, wherein a cell migration is performed, wherein the assay method comprises the following steps:

57

- providing cells which are capable of migration in a first part of a device, wherein the cells express MIF,
- adding the anti-(ox)MIF antibody to be determined, to a second part of the device, which is configured to be in a connection with the first part of the device which allows cell migration and diffusion, and
- calculating inhibition of migration, and
- wherein no (ox) MIF is added to the device.

2. Assay method according to claim 1, wherein the anti-MIF antibodies are anti-oxMIF antibodies, preferably wherein the antibodies are selected from the group consisting of RAB0 (as identified by SEQ ID NOs:3 and 7), RAB4 (as identified by SEQ ID NOs:2 and 6), RAB9 (as identified by SEQ ID NOs:1 and 5), RAM0 (as identified by SEQ ID NOs:9 and 10), RAM4 (as identified by SEQ ID NOs:13 and 14)and/or RAM9 (as identified by SEQ ID NOs:11 and 12), very preferred RAM9.

3. Assay method according to claim 1 or 2, wherein the device comprises an upper and a lower chamber, which are connected via a separating membrane with pores and in that the cells are added to the upper chamber and in that the antibodies are added to the lower chamber, preferably wherein the device is a two chamber cell migration assay (modified Boyden chamber assay), more preferably a Transwell cell migration assay device.

4. Assay method according to any one of the preceding claims, wherein the cells are cells which express (ox)MIF, preferably on their cell surface, preferably wherein the cells are cells from disease samples, more preferred wherein the cells are monocytic cells, preferably human monocytic cells, more preferred human immortalized monocytic cells, most preferred U937 cells, or THP-1 human monocytic cells or in an alternative embodiment rat NR 8383 monocytic cells.

5. Assay method according to any one of the preceding claims, wherein the cells are provided as a cell suspension in a suitable migration medium to allow migration.

6. Assay method according to claim 5, wherein the migration medium does not comprise proteins.

7. Assay method according to any one of the preceding claims, wherein the antibody is added in a non toxic biological buffer with a moderately weak acid and its conjugate base, preferably a glycine buffer, an N-substituted taurine buffer or a phosphate buffered saline (PBS) buffer, to the second part, e.g. lower chamber, of e.g. the Transwell® chamber.

8. Assay method according to any one of the preceding claims, wherein the antibody is added to have a final concentration in the assay of 0.01 -100 nM, preferably 0.02 to 50 nM, preferably 0.04-30 nM, preferably as a dilution series

9. Assay method according to any one of the preceding claims, wherein the assay, e.g. the Transwell® chamber, is incubated after addition of the cells and the antibody for 6-20 h, preferably 8-12 h, preferably at approximately 37°C, wherein the cells are U937 cells, and wherein the membrane has a pore size of approximately 5 $\mu$m.

10. Assay method according to any one of the preceding claims, wherein the migrated cells are counted, preferably after the above incubation step, and preferably in the lower chamber, whereupon information about the potency of the tested antibody can be obtained, preferably by calculating the half-maximal inhibiting antibody concentration (IC$_{50}$-value).

11. Assay method according to any one of the preceding claims, wherein the cells undergo from 10 to 48 h, preferably 8-16, preferably 10-12 h serum starvation before they are used in the assay.

**Patentansprüche**

1. Testverfahren zum Bestimmen der Wirkungsstärke von anti-MIF-Antikörpern, wobei eine Zellmigration durchgeführt wird, wobei das Testverfahren die folgenden Schritte umfasst:

- Bereitstellen von Zellen, die zur Migration fähig sind, in einem ersten Teil einer Vorrichtung, wobei die Zellen MIF exprimieren,
- Hinzugeben des zu bestimmenden anti-(ox)MIF-Antikörpers zu einem zweiten Teil der Vorrichtung, der dazu eingerichtet ist, in Verbindung mit dem ersten Teil der Vorrichtung zu stehen, was Zellmigration und Diffusion erlaubt, und
- Berechnen der Migrationsinhibition, und
- wobei kein (ox)MIF zur Vorrichtung hinzugegeben wird.

2. Testverfahren gemäß Anspruch 1, wobei die anti-MIF-Antikörper anti-oxMIF-Antikörper sind, wobei die Antikörper bevorzugt ausgewählt sind aus der Gruppe, bestehend aus RAB0 (wie durch SEQ ID NOs: 3 und 7 identifiziert), RAB4 (wie durch SEQ ID NOs: 2 und 6 identifiziert), RAB9 (wie durch SEQ ID NOs: 1 und 5 identifiziert), RAM0 (wie durch SEQ ID NOs: 9 und 10 identifiziert), RAM4 (wie durch SEQ ID NOs: 13 und 14 identifiziert) und/oder RAM9 (wie durch SEQ ID NOs: 11 und 12 identifiziert), sehr bevorzugt RAM9.

3. Testverfahren gemäß Anspruch 1 oder 2, wobei die Vorrichtung eine obere und eine untere Kammer umfasst, die durch eine trennende Membran mit Poren verbunden sind, und in dem die Zellen der oberen Kammer hinzugefügt werden und in dem die Antikörper der unteren Kammer hinzugefügt werden, wobei die Vorrichtung bevorzugt eine Zweikammer-Zellmigrations-Test-(modifizierte Boyden-Kammer-Test-), bevorzugter eine Transwell-Zellmigrations-Test-Vorrichtung ist.

4. Testverfahren gemäß einem der vorhergehenden Ansprüche, wobei die Zellen Zellen sind, die (ox)MIF exprimieren, bevorzugt auf ihrer Zelloberfläche, wobei die Zellen bevorzugt Zellen aus Krankheitsproben sind, wobei die Zellen bevorzugter Monozyten sind, bevorzugt menschliche Monozyten, bevorzugter menschliche immortalisierte Monozyten, besonders bevorzugt U937 Zellen oder menschliche THP-1 Monozyten oder in einer alternativen Ausführungsform NR 8383 Ratten-Monozyten.

5. Testverfahren gemäß einem der vorhergehenden Ansprüche, wobei die Zellen als eine Zellsuspension in einem geeigneten Migrationsmedium bereitgestellt werden, um Migration zu erlauben.

6. Testverfahren gemäß Anspruch 5, wobei das Migrationsmedium keine Proteine umfasst.

7. Testverfahren gemäß einem der vorhergehenden Ansprüche, wobei der Antikörper in einem nicht-toxischen biologischen Puffer mit einer moderat-schwachen Säure und ihrer konjugierten Base, bevorzugt einem Glycin-Puffer, einem N-substituiertes-Taurin-Puffer oder einem phosphatgepufferte-Salzlösungs- (PBS-)Puffer, zum zweiten Teil, z.B. der unteren Kammer von z.B. der Transwell®-Kammer hinzugefügt wird.

8. Testverfahren gemäß einem der vorhergehenden Ansprüche, wobei der Antikörper so hinzugegeben wird, dass er eine Endkonzentration im Test von 0,01 - 100 nM, bevorzugt 0,02 bis 50 nM, bevorzugt 0,04-30 nM, aufweist, bevorzugt als Verdünnungsreihe.

9. Testverfahren gemäß einem der vorhergehenden Ansprüche, wobei der Test, z.B. die Transwell®-Kammer, nach Zugabe der Zellen und des Antikörpers für 6-20 h, bevorzugt 8-12 h, inkubiert wird, bevorzugt bei ungefähr 37°C, wobei die Zellen U937 Zellen sind und wobei die Membran eine Porengröße von ungefähr 5 μm aufweist.

10. Testverfahren gemäß einem der vorhergehenden Ansprüche, wobei die migrierten Zellen gezählt werden, bevorzugt nach dem obigen Inkubationsschritt, und bevorzugt in der unteren Kammer, woraufhin Auskunft über die Wirkungsstärke des getesteten Antikörpers erhalten werden kann, bevorzugt durch das Berechnen der halbmaximalen inhibierenden Antikörperkonzentration ($IC_{50}$-Wert).

11. Testverfahren gemäß einem der vorhergehenden Ansprüche, wobei die Zellen 10 bis 48 h, bevorzugt 8-16, bevorzugt 10-12 h, einem Serumentzug unterzogen werden, bevor sie im Test verwendet werden.

## Revendications

1. Procédé de dosage pour déterminer l'activité d'anticorps anti-MIF, dans lequel une migration cellulaire est effectuée, dans lequel le procédé de dosage comprend les étapes suivantes :

   - la fourniture de cellules qui sont capables de migrer dans une première partie d'un dispositif, dans lequel les cellules expriment MIF,
   - l'ajout de l'anticorps anti-(ox)MIF à déterminer, à une seconde partie du dispositif, qui est configurée pour être raccordée à la première partie du dispositif qui permet la migration et la diffusion de cellules, et

- le calcul d'inhibition de migration, et
- dans lequel aucun (ox) MIF n'est ajouté au dispositif.

2. Procédé de dosage selon la revendication 1, dans lequel les anticorps anti-MIF sont des anticorps anti-oxMIF, de préférence dans lequel les anticorps sont choisis dans le groupe constitué de RAB0 (comme identifié par SEQ ID NO : 3 et 7), RAB4 (comme identifié par SEQ ID NO : 2 et 6), RAB9 (comme identifié par SEQ ID NO : 1 et 5), RAM0 (comme identifié par SEQ ID NO : 9 et 10), RAM4 (comme identifié par SEQ ID NO : 13 et 14) et/ou RAM9 (comme identifié par SEQ ID NO : 11 et 12), de manière particulièrement préférée RAM9.

3. Procédé de dosage selon la revendication 1 ou 2, dans lequel le dispositif comprend une chambre supérieure et une chambre inférieure, qui sont raccordées par l'intermédiaire d'une membrane de séparation ayant des pores, et en ce que les cellules sont ajoutées à la chambre supérieure et en ce que les anticorps sont ajoutés à la chambre inférieure, de préférence dans lequel le dispositif est un dosage de migration cellulaire à deux chambres (dosage Boyden chambre modifié), de manière davantage préférée un dispositif de dosage de migration cellulaire Transwell.

4. Procédé de dosage selon l'une quelconque des revendications précédentes, dans lequel les cellules sont des cellules qui expriment (ox)MIF, de préférence sur leur surface cellulaire, de préférence dans lequel les cellules sont des cellules provenant d'échantillons de maladie, de manière davantage préférée dans lequel les cellules sont des cellules monocytaires, de préférence des cellules monocytaires humaines, de manière davantage préférée des cellules monocytaires humaines immortalisées, de manière préférée entre toutes des cellules U937, ou des cellules monocytaires humaines THP-1, ou dans un mode de réalisation alternatif des cellules monocytaires NR 8383 de rat.

5. Procédé de dosage selon l'une quelconque des revendications précédentes, dans lequel les cellules sont fournies sous la forme d'une suspension cellulaire dans un milieu de migration approprié pour permettre une migration.

6. Procédé de dosage selon la revendication 5, dans lequel le milieu de migration ne comprend pas de protéines.

7. Procédé de dosage selon l'une quelconque des revendications précédentes, dans lequel l'anticorps est ajouté à un tampon biologique non toxique avec un acide modérément faible et sa base conjuguée, de préférence un tampon à la glycine, un tampon à taurine N-substituée ou un tampon salin tamponné au phosphate (PBS), à la seconde partie, par exemple la chambre inférieure, de, par exemple, la chambre Transwell®.

8. Procédé de dosage selon l'une quelconque des revendications précédentes, dans lequel l'anticorps est ajouté pour avoir une concentration finale dans le dosage de 0,01 à 100 nM, de préférence de 0,02 à 50 nM, de préférence de 0,04 à 30 nM, de préférence sous la forme d'une série de dilution.

9. Procédé de dosage selon l'une quelconque des revendications précédentes, dans lequel le dosage, par exemple la chambre Transwell®, est mis en incubation après l'ajout des cellules et de l'anticorps pendant 6 à 20 heures, préférence 8 à 12 heures, de préférence à environ 37 °C, dans lequel les cellules sont des cellules U937, et dans lequel la membrane présente une taille de pore d'environ 5 $\mu$m.

10. Procédé de dosage selon l'une quelconque des revendications précédentes, dans lequel les cellules qui ont migré sont comptées, de préférence après l'étape d'incubation ci-dessus, et de préférence dans la chambre inférieure, après quoi des informations concernant l'activité de l'anticorps testé peuvent être obtenues, de préférence en calculant la concentration à mi-hauteur en anticorps inhibant (valeur de $IC_{50}$).

11. Procédé de dosage selon l'une quelconque des revendications précédentes, dans lequel les cellules sont privées de sérum pendant 10 à 48 heures, de préférence pendant 8 à 16 heures, de préférence pendant 10 à 12 heures avant qu'elles soient utilisées dans le dosage.

Figure 1:

Figure 2:

Grey line: isotype control
Black line: RAM9

Figure 3:

EP 2 929 348 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 6645493 B
- US 200310235584 A
- EP 2231707 A
- WO 2009086920 A

### Non-patent literature cited in the description

- **BLOOM et al.** *Science,* 1966, vol. 153, 80-2
- **DAVID et al.** *PNAS,* 1966, vol. 56, 72-7
- **WEISER et al.** *PNAS,* 1989, vol. 86, 7522-6
- **SUN et al.** *PNAS,* 1996, vol. 93, 5191-5196
- **CALANDRA et al.** *Nature,* 1995, vol. 377, 68-71
- **BAUGH et al.** *Crit Care Med,* 2002, vol. 30, 27-35
- **MITCHELL, R.A.** *Cellular Signalling,* 2004, vol. 16 (1), 13-19
- **LUE, H. et al.** *Oncogene,* 2007, vol. 26 (35), 5046-59
- **NISHIHIRA et al.** *J Interferon Cytokine Res.,* 2000, vol. 20, 751-62
- **SHIMIZU et al.** *FEBS Lett.,* 1996, vol. 381, 199-202
- **KAWAGUCHI et al.** *Leukoc. Biol.,* 1986, vol. 39, 223-232
- **WEISER et al.** *Cell. Immunol.,* 1985, vol. 90, 167-78
- **CALANDRA et al.** *J. Inflamm.,* 1995, vol. 47, 39-51
- **HAGEMANN et al.** Journal of Immunology. The American Association of Immunologists, 01 July 2005, vol. 175, 1197-1205
- **GALAT et al.** *Eur. J. Biochem,* 1994, vol. 224, 417-21
- **WATARAI et al.** *PNAS,* 2000, vol. 97, 13251-6
- **BEMHAGEN et al.** *Nature Medicine,* 2007
- **SAMBROOK et al.** Molecular Cloning: A Laboratory Manual. Cold Spring Harbor Laboratory Press, 1989
- **AUSUBEL et al.** Current Protocols in Molecular Biology. Greene Publishing Associates, 1992
- **HARLOW ; LANE.** Antibodies: A Laboratory Manual. Cold Spring Harbor Laboratory Press, 1990
- Fundamental Immunology. Raven Press, 1989
- **KABAT.** Sequences of Proteins of Immunological Interest. National Institutes of Health, 1987
- **CHOTHIA et al.** *J. Mol. Biol.,* 1987, vol. 196, 901-917
- **CHOTHIA et al.** *Nature,* 1989, vol. 342, 878-883
- **ANGAL et al.** *Mol Immunol.,* 1993, vol. 30, 105-108

63

SEQUENCE LISTING

<110> Baxter Healthcare S.A. Baxter International Inc.

<120> ANTI-MIF ANTIBODY CELL MIGRATION ASSAY

<130> 162 869

<160> 14

<170> PatentIn version 3.5

<210> 1
<211> 214
<212> PRT
<213> Artificial Sequence

<220>
<223> Light chain of RAB9

<400> 1

```
Asp Ile Gln Met Thr Gln Ser Pro Ser Ser Leu Ser Ala Ser Val Gly
1               5                   10                  15

Asp Arg Val Thr Ile Thr Cys Arg Ser Ser Gln Arg Ile Met Thr Tyr
            20                  25                  30

Leu Asn Trp Tyr Gln Gln Lys Pro Gly Lys Ala Pro Lys Leu Leu Ile
        35                  40                  45

Phe Val Ala Ser His Ser Gln Ser Gly Val Pro Ser Arg Phe Arg Gly
    50                  55                  60

Ser Gly Ser Glu Thr Asp Phe Thr Leu Thr Ile Ser Gly Leu Gln Pro
65                  70                  75                  80

Glu Asp Ser Ala Thr Tyr Tyr Cys Gln Gln Ser Phe Trp Thr Pro Leu
                85                  90                  95

Thr Phe Gly Gly Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
        210
```

<210> 2
<211> 214
<212> PRT
<213> Artificial Sequence

<220>

<223> Light chain of RAB4

<400> 2

```
Asp Ile Gln Met Thr Gln Ser Pro Gly Thr Leu Ser Leu Ser Pro Gly
1               5                   10                  15

Glu Arg Ala Thr Leu Ser Cys Arg Ala Ser Gln Gly Val Ser Ser Ser
            20                  25                  30

Ser Leu Ala Trp Tyr Gln Gln Lys Pro Gly Gln Ala Pro Arg Leu Leu
            35                  40                  45

Ile Tyr Gly Thr Ser Ser Arg Ala Thr Gly Ile Pro Asp Arg Phe Ser
        50                  55                  60

Gly Ser Ala Ser Gly Thr Asp Phe Thr Leu Thr Ile Ser Arg Leu Gln
65                  70                  75                  80

Pro Glu Asp Phe Ala Val Tyr Tyr Cys Gln Gln Tyr Gly Arg Ser Leu
                85                  90                  95

Thr Phe Gly Gly Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
            115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
            165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
            195                 200                 205

Phe Asn Arg Gly Glu Cys
            210
```

<210> 3
<211> 214
<212> PRT
<213> Artificial Sequence

<220>
<223> Light chain of RAB0

<400> 3

```
Asp Ile Gln Met Thr Gln Ser Pro Gly Thr Leu Ser Leu Ser Pro Gly
1               5                   10                  15

Glu Arg Ala Thr Leu Ser Cys Arg Ala Ser Gln Gly Val Ser Ser Ser
            20                  25                  30

Ser Leu Ala Trp Tyr Gln Gln Lys Pro Gly Gln Ala Pro Arg Leu Leu
            35                  40                  45

Ile Tyr Gly Thr Ser Ser Arg Ala Thr Gly Ile Pro Asp Arg Phe Ser
        50                  55                  60

Gly Ser Ala Ser Gly Thr Asp Phe Thr Leu Thr Ile Ser Arg Leu Gln
65                  70                  75                  80

Pro Glu Asp Phe Ala Val Tyr Tyr Cys Gln Gln Tyr Gly Arg Ser Leu
                85                  90                  95

Thr Phe Gly Gly Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
            115                 120                 125

Thr Ala Ser Val Val Cys Leu Leu Asn Asn Phe Tyr Pro Arg Glu Ala
    130                 135                 140
```

EP 2 929 348 B1

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
                180                 185                 190

Ala Cys Glu Val Thr His Gln Gly Leu Ser Ser Pro Val Thr Lys Ser
        195                 200                 205

Phe Asn Arg Gly Glu Cys
        210

<210> 4
<211> 214
<212> PRT
<213> Artificial Sequence

<220>
<223> Light chain of RAB2

<400> 4

Asp Ile Gln Met Thr Gln Ser Pro Val Thr Leu Ser Leu Ser Pro Gly
1               5                   10                  15

Glu Arg Ala Thr Leu Ser Cys Arg Ala Ser Gln Ser Val Arg Ser Ser
            20                  25                  30

Tyr Leu Ala Trp Tyr Gln Gln Lys Pro Gly Gln Thr Pro Arg Leu Leu
            35                  40                  45

Ile Tyr Gly Ala Ser Asn Arg Ala Thr Gly Ile Pro Asp Arg Phe Ser
        50                  55                  60

Gly Ser Gly Ser Gly Thr Asp Phe Thr Leu Thr Ile Ser Arg Leu Glu
65                  70                  75                  80

Pro Glu Asp Phe Ala Val Tyr Tyr Cys Gln Gln Tyr Gly Asn Ser Leu
                85                  90                  95

Thr Phe Gly Gly Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
            115                 120                 125

37

```
Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
    145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
        210
```

<210> 5
<211> 445
<212> PRT
<213> Artificial Sequence

<220>
<223> Heavy chain of RAB9

<400> 5

```
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Ser Met Asn Trp Val Arg Gln Ala Pro Gly Lys Gly Leu Glu Trp Val
            35                  40                  45

Ser Ser Ile Gly Ser Ser Gly Gly Thr Thr Tyr Tyr Ala Asp Ser Val
        50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Gly Ser Gln Trp Leu Tyr Gly Met Asp Val Trp Gly Gln Gly Thr
            100                 105                 110
```

Thr Val Thr Val Ser Ser Ala Ser Thr Lys Gly Pro Ser Val Phe Pro
        115             120             125

Leu Ala Pro Cys Ser Arg Ser Thr Ser Glu Ser Thr Ala Ala Leu Gly
        130             135             140

Cys Leu Val Lys Asp Tyr Phe Pro Glu Pro Val Thr Val Ser Trp Asn
145             150             155             160

Ser Gly Ala Leu Thr Ser Gly Val His Thr Phe Pro Ala Val Leu Gln
                165             170             175

Ser Ser Gly Leu Tyr Ser Leu Ser Ser Val Val Thr Val Pro Ser Ser
        180             185             190

Ser Leu Gly Thr Lys Thr Tyr Thr Cys Asn Val Asp His Lys Pro Ser
        195             200             205

Asn Thr Lys Val Asp Lys Arg Val Glu Ser Lys Tyr Gly Pro Pro Cys
210             215             220

Pro Pro Cys Pro Ala Pro Glu Phe Leu Gly Gly Pro Ser Val Phe Leu
225             230             235             240

Phe Pro Pro Lys Pro Lys Asp Thr Leu Met Ile Ser Arg Thr Pro Glu
                245             250             255

Val Thr Cys Val Val Val Asp Val Ser Gln Glu Asp Pro Glu Val Gln
                260             265             270

Phe Asn Trp Tyr Val Asp Gly Val Glu Val His Asn Ala Lys Thr Lys
        275             280             285

Pro Arg Glu Glu Gln Phe Asn Ser Thr Tyr Arg Val Val Ser Val Leu
        290             295             300

Thr Val Leu His Gln Asp Trp Leu Asn Gly Lys Glu Tyr Lys Cys Lys
305             310             315             320

Val Ser Asn Lys Gly Leu Pro Ser Ser Ile Glu Lys Thr Ile Ser Lys
                325             330             335

Ala Lys Gly Gln Pro Arg Glu Pro Gln Val Tyr Thr Leu Pro Pro Ser
                340             345             350

Gln Glu Glu Met Thr Lys Asn Gln Val Ser Leu Thr Cys Leu Val Lys
                355             360             365

```
        Gly Phe Tyr Pro Ser Asp Ile Ala Val Glu Trp Glu Ser Asn Gly Gln
            370             375             380

        Pro Glu Asn Asn Tyr Lys Thr Thr Pro Pro Val Leu Asp Ser Asp Gly
        385             390             395                 400

        Ser Phe Phe Leu Tyr Ser Arg Leu Thr Val Asp Lys Ser Arg Trp Gln
                        405             410                 415

        Glu Gly Asn Val Phe Ser Cys Ser Val Met His Glu Ala Leu His Asn
                        420             425             430

        His Tyr Thr Gln Lys Ser Leu Ser Leu Ser Leu Gly Lys
                        435             440             445
```

<210> 6
<211> 454
<212> PRT
<213> Artificial Sequence

<220>
<223> Heavy chain of RAB4

<400> 6

```
        Glu Val Gln Leu Leu Glu Ser Gly Gly Gly Leu Val Gln Pro Gly Gly
        1               5               10                  15

        Ser Leu Arg Leu Ser Cys Ala Ala Ser Gly Phe Thr Phe Ser Ile Tyr
                        20              25                  30

        Ala Met Asp Trp Val Arg Gln Ala Pro Gly Lys Gly Leu Glu Trp Val
                        35              40                  45

        Ser Gly Ile Val Pro Ser Gly Gly Phe Thr Lys Tyr Ala Asp Ser Val
                        50              55                  60

        Lys Gly Arg Phe Thr Ile Ser Arg Asp Asn Ser Lys Asn Thr Leu Tyr
        65                      70              75                  80

        Leu Gln Met Asn Ser Leu Arg Ala Glu Asp Thr Ala Val Tyr Tyr Cys
                        85              90                  95

        Ala Arg Val Asn Val Ile Ala Val Ala Gly Thr Gly Tyr Tyr Tyr Tyr
                        100             105                 110

        Gly Met Asp Val Trp Gly Gln Gly Thr Thr Val Thr Val Ser Ser Ala
                        115             120                 125
```

```
Ser Thr Lys Gly Pro Ser Val Phe Pro Leu Ala Pro Cys Ser Arg Ser
    130             135             140

Thr Ser Glu Ser Thr Ala Ala Leu Gly Cys Leu Val Lys Asp Tyr Phe
    145             150             155             160

Pro Glu Pro Val Thr Val Ser Trp Asn Ser Gly Ala Leu Thr Ser Gly
                165             170             175

Val His Thr Phe Pro Ala Val Leu Gln Ser Ser Gly Leu Tyr Ser Leu
            180             185             190

Ser Ser Val Val Thr Val Pro Ser Ser Ser Leu Gly Thr Lys Thr Tyr
        195             200             205

Thr Cys Asn Val Asp His Lys Pro Ser Asn Thr Lys Val Asp Lys Arg
    210             215             220

Val Glu Ser Lys Tyr Gly Pro Pro Cys Pro Pro Cys Pro Ala Pro Glu
225             230             235             240

Phe Leu Gly Gly Pro Ser Val Phe Leu Phe Pro Pro Lys Pro Lys Asp
            245             250             255

Thr Leu Met Ile Ser Arg Thr Pro Glu Val Thr Cys Val Val Val Asp
            260             265             270

Val Ser Gln Glu Asp Pro Glu Val Gln Phe Asn Trp Tyr Val Asp Gly
        275             280             285

Val Glu Val His Asn Ala Lys Thr Lys Pro Arg Glu Glu Gln Phe Asn
    290             295             300

Ser Thr Tyr Arg Val Val Ser Val Leu Thr Val Leu His Gln Asp Trp
305             310             315             320

Leu Asn Gly Lys Glu Tyr Lys Cys Lys Val Ser Asn Lys Gly Leu Pro
            325             330             335

Ser Ser Ile Glu Lys Thr Ile Ser Lys Ala Lys Gly Gln Pro Arg Glu
            340             345             350

Pro Gln Val Tyr Thr Leu Pro Pro Ser Gln Glu Glu Met Thr Lys Asn
        355             360             365

Gln Val Ser Leu Thr Cys Leu Val Lys Gly Phe Tyr Pro Ser Asp Ile
    370             375             380
```

41

```
Ala Val Glu Trp Glu Ser Asn Gly Gln Pro Glu Asn Asn Tyr Lys Thr
385                 390                 395                 400

Thr Pro Pro Val Leu Asp Ser Asp Gly Ser Phe Phe Leu Tyr Ser Arg
                405                 410                 415

Leu Thr Val Asp Lys Ser Arg Trp Gln Glu Gly Asn Val Phe Ser Cys
                420                 425                 430

Ser Val Met His Glu Ala Leu His Asn His Tyr Thr Gln Lys Ser Leu
                435                 440                 445

Ser Leu Ser Leu Gly Lys
                450
```

<210> 7
<211> 454
<212> PRT
<213> Artificial Sequence

<220>
<223> Heavy chain of RAB0

<400> 7

```
Glu Val Gln Leu Leu Glu Ser Gly Gly Gly Leu Val Gln Pro Gly Gly
1                 5                   10                  15

Ser Leu Arg Leu Ser Cys Ala Ala Ser Gly Phe Thr Phe Ser Trp Tyr
                20                  25                  30

Ala Met Asp Trp Val Arg Gln Ala Pro Gly Lys Gly Leu Glu Trp Val
                35                  40                  45

Ser Gly Ile Tyr Pro Ser Gly Gly Arg Thr Lys Tyr Ala Asp Ser Val
                50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Val Asn Val Ile Ala Val Ala Gly Thr Gly Tyr Tyr Tyr Tyr
                100                 105                 110

Gly Met Asp Val Trp Gly Gln Gly Thr Thr Val Thr Val Ser Ser Ala
                115                 120                 125
```

```
Ser Thr Lys Gly Pro Ser Val Phe Pro Leu Ala Pro Cys Ser Arg Ser
    130             135             140

Thr Ser Glu Ser Thr Ala Ala Leu Gly Cys Leu Val Lys Asp Tyr Phe
    145             150             155             160

Pro Glu Pro Val Thr Val Ser Trp Asn Ser Gly Ala Leu Thr Ser Gly
                165             170             175

Val His Thr Phe Pro Ala Val Leu Gln Ser Ser Gly Leu Tyr Ser Leu
            180             185             190

Ser Ser Val Val Thr Val Pro Ser Ser Ser Leu Gly Thr Lys Thr Tyr
            195             200             205

Thr Cys Asn Val Asp His Lys Pro Ser Asn Thr Lys Val Asp Lys Arg
    210             215             220

Val Glu Ser Lys Tyr Gly Pro Pro Cys Pro Pro Cys Pro Ala Pro Glu
225             230             235             240

Phe Leu Gly Gly Pro Ser Val Phe Leu Phe Pro Pro Lys Pro Lys Asp
            245             250             255

Thr Leu Met Ile Ser Arg Thr Pro Glu Val Thr Cys Val Val Val Asp
            260             265             270

Val Ser Gln Glu Asp Pro Glu Val Gln Phe Asn Trp Tyr Val Asp Gly
        275             280             285

Val Glu Val His Asn Ala Lys Thr Lys Pro Arg Glu Glu Gln Phe Asn
    290             295             300

Ser Thr Tyr Arg Val Val Ser Val Leu Thr Val Leu His Gln Asp Trp
305             310             315             320

Leu Asn Gly Lys Glu Tyr Lys Cys Lys Val Ser Asn Lys Gly Leu Pro
            325             330             335

Ser Ser Ile Glu Lys Thr Ile Ser Lys Ala Lys Gly Gln Pro Arg Glu
            340             345             350

Pro Gln Val Tyr Thr Leu Pro Pro Ser Gln Glu Glu Met Thr Lys Asn
        355             360             365

Gln Val Ser Leu Thr Cys Leu Val Lys Gly Phe Tyr Pro Ser Asp Ile
    370             375             380
```

```
        Ala Val Glu Trp Glu Ser Asn Gly Gln Pro Glu Asn Asn Tyr Lys Thr
        385             390             395                     400


        Thr Pro Pro Val Leu Asp Ser Asp Gly Ser Phe Phe Leu Tyr Ser Arg
                        405             410                 415


        Leu Thr Val Asp Lys Ser Arg Trp Gln Glu Gly Asn Val Phe Ser Cys
                    420             425                 430


        Ser Val Met His Glu Ala Leu His Asn His Tyr Thr Gln Lys Ser Leu
                    435             440             445


        Ser Leu Ser Leu Gly Lys
            450
```

<210> 8
<211> 454
<212> PRT
<213> Artificial Sequence

<220>
<223> Heavy chain of RAB2

<400> 8

```
        Glu Val Gln Leu Leu Glu Ser Gly Gly Gly Leu Val Gln Pro Gly Gly
        1               5                   10                  15


        Ser Leu Arg Leu Ser Cys Ala Ala Ser Gly Phe Thr Phe Ser Ile Tyr
                    20              25                  30


        Ala Met Asp Trp Val Arg Gln Ala Pro Gly Lys Gly Leu Glu Trp Val
                    35              40              45


        Ser Gly Ile Val Pro Ser Gly Gly Phe Thr Lys Tyr Ala Asp Ser Val
            50              55              60


        Lys Gly Arg Phe Thr Ile Ser Arg Asp Asn Ser Lys Asn Thr Leu Tyr
        65              70              75                      80


        Leu Gln Met Asn Ser Leu Arg Ala Glu Asp Thr Ala Val Tyr Tyr Cys
                        85              90                      95


        Ala Arg Val Asn Val Ile Ala Val Ala Gly Thr Gly Tyr Tyr Tyr Tyr
                    100             105                 110


        Gly Met Asp Val Trp Gly Gln Gly Thr Thr Val Thr Val Ser Ser Ala
                    115             120                 125
```

Ser Thr Lys Gly Pro Ser Val Phe Pro Leu Ala Pro Cys Ser Arg Ser
130 135 140

Thr Ser Glu Ser Thr Ala Ala Leu Gly Cys Leu Val Lys Asp Tyr Phe
145 150 155 160

Pro Glu Pro Val Thr Val Ser Trp Asn Ser Gly Ala Leu Thr Ser Gly
165 170 175

Val His Thr Phe Pro Ala Val Leu Gln Ser Ser Gly Leu Tyr Ser Leu
180 185 190

Ser Ser Val Val Thr Val Pro Ser Ser Ser Leu Gly Thr Lys Thr Tyr
195 200 205

Thr Cys Asn Val Asp His Lys Pro Ser Asn Thr Lys Val Asp Lys Arg
210 215 220

Val Glu Ser Lys Tyr Gly Pro Pro Cys Pro Pro Cys Pro Ala Pro Glu
225 230 235 240

Phe Leu Gly Gly Pro Ser Val Phe Leu Phe Pro Pro Lys Pro Lys Asp
245 250 255

Thr Leu Met Ile Ser Arg Thr Pro Glu Val Thr Cys Val Val Val Asp
260 265 270

Val Ser Gln Glu Asp Pro Glu Val Gln Phe Asn Trp Tyr Val Asp Gly
275 280 285

Val Glu Val His Asn Ala Lys Thr Lys Pro Arg Glu Glu Gln Phe Asn
290 295 300

Ser Thr Tyr Arg Val Val Ser Val Leu Thr Val Leu His Gln Asp Trp
305 310 315 320

Leu Asn Gly Lys Glu Tyr Lys Cys Lys Val Ser Asn Lys Gly Leu Pro
325 330 335

Ser Ser Ile Glu Lys Thr Ile Ser Lys Ala Lys Gly Gln Pro Arg Glu
340 345 350

Pro Gln Val Tyr Thr Leu Pro Pro Ser Gln Glu Glu Met Thr Lys Asn
355 360 365

Gln Val Ser Leu Thr Cys Leu Val Lys Gly Phe Tyr Pro Ser Asp Ile
370 375 380

```
Ala Val Glu Trp Glu Ser Asn Gly Gln Pro Glu Asn Asn Tyr Lys Thr
385             390             395             400

Thr Pro Pro Val Leu Asp Ser Asp Gly Ser Phe Phe Leu Tyr Ser Arg
                405             410             415

Leu Thr Val Asp Lys Ser Arg Trp Gln Glu Gly Asn Val Phe Ser Cys
                420             425             430

Ser Val Met His Glu Ala Leu His Asn His Tyr Thr Gln Lys Ser Leu
                435             440             445

Ser Leu Ser Leu Gly Lys
                450
```

<210> 9
<211> 457
<212> PRT
<213> Artificial Sequence

<220>
<223> RAM0hc

<400> 9

```
Glu Val Gln Leu Leu Glu Ser Gly Gly Gly Leu Val Gln Pro Gly Gly
1               5               10              15

Ser Leu Arg Leu Ser Cys Ala Ala Ser Gly Phe Thr Phe Ser Trp Tyr
                20              25              30

Ala Met Asp Trp Val Arg Gln Ala Pro Gly Lys Gly Leu Glu Trp Val
                35              40              45

Ser Gly Ile Tyr Pro Ser Gly Gly Arg Thr Lys Tyr Ala Asp Ser Val
                50              55              60

Lys Gly Arg Phe Thr Ile Ser Arg Asp Asn Ser Lys Asn Thr Leu Tyr
65              70              75              80

Leu Gln Met Asn Ser Leu Arg Ala Glu Asp Thr Ala Val Tyr Tyr Cys
                85              90              95

Ala Arg Val Asn Val Ile Ala Val Ala Gly Thr Gly Tyr Tyr Tyr Tyr
                100             105             110

Gly Met Asp Val Trp Gly Gln Gly Thr Thr Val Thr Val Ser Ser Ala
                115             120             125
```

Ser Thr Lys Gly Pro Ser Val Phe Pro Leu Ala Pro Ser Ser Lys Ser
    130             135             140

Thr Ser Gly Gly Thr Ala Ala Leu Gly Cys Leu Val Lys Asp Tyr Phe
    145             150             155             160

Pro Glu Pro Val Thr Val Ser Trp Asn Ser Gly Ala Leu Thr Ser Gly
                165             170             175

Val His Thr Phe Pro Ala Val Leu Gln Ser Ser Gly Leu Tyr Ser Leu
            180             185             190

Ser Ser Val Val Thr Val Pro Ser Ser Ser Leu Gly Thr Gln Thr Tyr
        195             200             205

Ile Cys Asn Val Asn His Lys Pro Ser Asn Thr Lys Val Asp Lys Arg
    210             215             220

Val Glu Pro Lys Ser Cys Asp Lys Thr His Thr Cys Pro Pro Cys Pro
225             230             235             240

Ala Pro Glu Leu Leu Gly Gly Pro Ser Val Phe Leu Phe Pro Pro Lys
            245             250             255

Pro Lys Asp Thr Leu Met Ile Ser Arg Thr Pro Glu Val Thr Cys Val
            260             265             270

Val Val Asp Val Ser His Glu Asp Pro Glu Val Lys Phe Asn Trp Tyr
        275             280             285

Val Asp Gly Val Glu Val His Asn Ala Lys Thr Lys Pro Arg Glu Glu
    290             295             300

Gln Tyr Asn Ser Thr Tyr Arg Val Val Ser Val Leu Thr Val Leu His
305             310             315             320

Gln Asp Trp Leu Asn Gly Lys Glu Tyr Lys Cys Lys Val Ser Asn Lys
            325             330             335

Ala Leu Pro Ala Pro Ile Glu Lys Thr Ile Ser Lys Ala Lys Gly Gln
            340             345             350

Pro Arg Glu Pro Gln Val Tyr Thr Leu Pro Pro Ser Arg Glu Glu Met
        355             360             365

Thr Lys Asn Gln Val Ser Leu Thr Cys Leu Val Lys Gly Phe Tyr Pro
    370             375             380

```
        Ser Asp Ile Ala Val Glu Trp Glu Ser Asn Gly Gln Pro Glu Asn Asn
        385             390             395             400


        Tyr Lys Thr Thr Pro Pro Val Leu Asp Ser Asp Gly Ser Phe Phe Leu
                        405             410             415


        Tyr Ser Lys Leu Thr Val Asp Lys Ser Arg Trp Gln Gln Gly Asn Val
                    420             425             430


        Phe Ser Cys Ser Val Met His Glu Ala Leu His Asn His Tyr Thr Gln
                    435             440             445


        Lys Ser Leu Ser Leu Ser Pro Gly Lys
            450             455
```

<210> 10
<211> 214
<212> PRT
<213> Artificial Sequence

<220>
<223> RAM0lc

<400> 10

```
        Asp Ile Gln Met Thr Gln Ser Pro Gly Thr Leu Ser Leu Ser Pro Gly
        1               5               10              15


        Glu Arg Ala Thr Leu Ser Cys Arg Ala Ser Gln Gly Val Ser Ser Ser
                    20              25              30


        Ser Leu Ala Trp Tyr Gln Gln Lys Pro Gly Gln Ala Pro Arg Leu Leu
                    35              40              45


        Ile Tyr Gly Thr Ser Ser Arg Ala Thr Gly Ile Pro Asp Arg Phe Ser
                50              55              60


        Gly Ser Ala Ser Gly Thr Asp Phe Thr Leu Thr Ile Ser Arg Leu Gln
        65              70              75              80


        Pro Glu Asp Phe Ala Val Tyr Tyr Cys Gln Gln Tyr Gly Arg Ser Leu
                        85              90              95


        Thr Phe Gly Gly Gly Thr Lys Val Glu Ile Lys Arg Thr Val Ala Ala
                    100             105             110


        Pro Ser Val Phe Ile Phe Pro Pro Ser Asp Glu Gln Leu Lys Ser Gly
                    115             120             125
```

```
Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145             150                 155                     160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210
```

<210> 11
<211> 448
<212> PRT
<213> Artificial Sequence

<220>
<223> RAM9hc

<400> 11

Glu Val Gln Leu Leu Glu Ser Gly Gly Gly Leu Val Gln Pro Gly Gly
1                   5                   10                  15

Ser Leu Arg Leu Ser Cys Ala Ala Ser Gly Phe Thr Phe Ser Ile Tyr
            20                  25                  30

Ser Met Asn Trp Val Arg Gln Ala Pro Gly Lys Gly Leu Glu Trp Val
            35                  40                  45

Ser Ser Ile Gly Ser Ser Gly Gly Thr Thr Tyr Tyr Ala Asp Ser Val
        50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Gly Ser Gln Trp Leu Tyr Gly Met Asp Val Trp Gly Gln Gly Thr
            100                 105                 110

Thr Val Thr Val Ser Ser Ala Ser Thr Lys Gly Pro Ser Val Phe Pro

50

|  |  |  | 115 |  |  |  | 120 |  |  |  | 125 |  |  |  |
|---|---|---|---|---|---|---|---|---|---|---|---|---|---|---|

Leu Ala Pro Ser Ser Lys Ser Thr Ser Gly Gly Thr Ala Ala Leu Gly
130       135             140

Cys Leu Val Lys Asp Tyr Phe Pro Glu Pro Val Thr Val Ser Trp Asn
145         150             155             160

Ser Gly Ala Leu Thr Ser Gly Val His Thr Phe Pro Ala Val Leu Gln
            165             170             175

Ser Ser Gly Leu Tyr Ser Leu Ser Ser Val Val Thr Val Pro Ser Ser
            180             185             190

Ser Leu Gly Thr Gln Thr Tyr Ile Cys Asn Val Asn His Lys Pro Ser
            195             200             205

Asn Thr Lys Val Asp Lys Arg Val Glu Pro Lys Ser Cys Asp Lys Thr
210             215             220

His Thr Cys Pro Pro Cys Pro Ala Pro Glu Leu Leu Gly Gly Pro Ser
225             230             235             240

Val Phe Leu Phe Pro Pro Lys Pro Lys Asp Thr Leu Met Ile Ser Arg
            245             250             255

Thr Pro Glu Val Thr Cys Val Val Val Asp Val Ser His Glu Asp Pro
            260             265             270

Glu Val Lys Phe Asn Trp Tyr Val Asp Gly Val Glu Val His Asn Ala
            275             280             285

Lys Thr Lys Pro Arg Glu Glu Gln Tyr Asn Ser Thr Tyr Arg Val Val
            290             295             300

Ser Val Leu Thr Val Leu His Gln Asp Trp Leu Asn Gly Lys Glu Tyr
305             310             315             320

Lys Cys Lys Val Ser Asn Lys Ala Leu Pro Ala Pro Ile Glu Lys Thr
            325             330             335

Ile Ser Lys Ala Lys Gly Gln Pro Arg Glu Pro Gln Val Tyr Thr Leu
            340             345             350

Pro Pro Ser Arg Glu Glu Met Thr Lys Asn Gln Val Ser Leu Thr Cys
            355             360             365

```
Leu Val Lys Gly Phe Tyr Pro Ser Asp Ile Ala Val Glu Trp Glu Ser
    370                 375                 380

Asn Gly Gln Pro Glu Asn Asn Tyr Lys Thr Thr Pro Pro Val Leu Asp
385                 390                 395                 400

Ser Asp Gly Ser Phe Phe Leu Tyr Ser Lys Leu Thr Val Asp Lys Ser
                405                 410                 415

Arg Trp Gln Gln Gly Asn Val Phe Ser Cys Ser Val Met His Glu Ala
                420                 425                 430

Leu His Asn His Tyr Thr Gln Lys Ser Leu Ser Leu Ser Pro Gly Lys
            435                 440                 445
```

<210> 12
<211> 214
<212> PRT
<213> Artificial Sequence

<220>
<223> RAM9lc

<400> 12

```
Asp Ile Gln Met Thr Gln Ser Pro Ser Ser Leu Ser Ala Ser Val Gly
1               5                   10                  15

Asp Arg Val Thr Ile Thr Cys Arg Ser Ser Gln Arg Ile Met Thr Tyr
            20                  25                  30

Leu Asn Trp Tyr Gln Gln Lys Pro Gly Lys Ala Pro Lys Leu Leu Ile
            35                  40                  45

Phe Val Ala Ser His Ser Gln Ser Gly Val Pro Ser Arg Phe Arg Gly
    50                  55                  60

Ser Gly Ser Glu Thr Asp Phe Thr Leu Thr Ile Ser Gly Leu Gln Pro
65                  70                  75                  80

Glu Asp Ser Ala Thr Tyr Tyr Cys Gln Gln Ser Phe Trp Thr Pro Leu
                85                  90                  95

Thr Phe Gly Gly Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
            115                 120                 125

Thr Ala Ser Val Val Cys Leu Leu Asn Asn Phe Tyr Pro Arg Glu Ala
        130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
        210
```

<210> 13
<211> 457
<212> PRT
<213> Artificial Sequence

53

<220>
<223> RAM4hc

<400> 13

```
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Ala Met Asp Trp Val Arg Gln Ala Pro Gly Lys Gly Leu Glu Trp Val
            35                  40                  45

Ser Gly Ile Val Pro Ser Gly Gly Phe Thr Lys Tyr Ala Asp Ser Val
        50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Val Asn Val Ile Ala Val Ala Gly Thr Gly Tyr Tyr Tyr Tyr
            100                 105                 110

Gly Met Asp Val Trp Gly Gln Gly Thr Thr Val Thr Val Ser Ser Ala
            115                 120                 125
```

```
Ser Thr Lys Gly Pro Ser Val Phe Pro Leu Ala Pro Ser Ser Lys Ser
    130                 135             140

Thr Ser Gly Gly Thr Ala Ala Leu Gly Cys Leu Val Lys Asp Tyr Phe
    145             150             155                 160

Pro Glu Pro Val Thr Val Ser Trp Asn Ser Gly Ala Leu Thr Ser Gly
                165             170             175

Val His Thr Phe Pro Ala Val Leu Gln Ser Ser Gly Leu Tyr Ser Leu
            180             185             190

Ser Ser Val Val Thr Val Pro Ser Ser Ser Leu Gly Thr Gln Thr Tyr
        195             200             205

Ile Cys Asn Val Asn His Lys Pro Ser Asn Thr Lys Val Asp Lys Arg
    210             215             220

Val Glu Pro Lys Ser Cys Asp Lys Thr His Thr Cys Pro Pro Cys Pro
225             230             235                 240

Ala Pro Glu Leu Leu Gly Gly Pro Ser Val Phe Leu Phe Pro Pro Lys
            245             250             255

Pro Lys Asp Thr Leu Met Ile Ser Arg Thr Pro Glu Val Thr Cys Val
            260             265             270

Val Val Asp Val Ser His Glu Asp Pro Glu Val Lys Phe Asn Trp Tyr
        275             280             285

Val Asp Gly Val Glu Val His Asn Ala Lys Thr Lys Pro Arg Glu Glu
        290             295             300

Gln Tyr Asn Ser Thr Tyr Arg Val Val Ser Val Leu Thr Val Leu His
305             310             315                 320

Gln Asp Trp Leu Asn Gly Lys Glu Tyr Lys Cys Lys Val Ser Asn Lys
                325             330             335

Ala Leu Pro Ala Pro Ile Glu Lys Thr Ile Ser Lys Ala Lys Gly Gln
            340             345             350

Pro Arg Glu Pro Gln Val Tyr Thr Leu Pro Pro Ser Arg Glu Glu Met
        355             360             365

Thr Lys Asn Gln Val Ser Leu Thr Cys Leu Val Lys Gly Phe Tyr Pro
```

|  | 370 |  |  |  |  | 375 |  |  |  |  | 380 |  |  |  |
|---|---|---|---|---|---|---|---|---|---|---|---|---|---|---|

Ser Asp Ile Ala Val Glu Trp Glu Ser Asn Gly Gln Pro Glu Asn Asn
385             390             395                 400

Tyr Lys Thr Thr Pro Pro Val Leu Asp Ser Asp Gly Ser Phe Phe Leu
            405             410             415

Tyr Ser Lys Leu Thr Val Asp Lys Ser Arg Trp Gln Gln Gly Asn Val
        420             425             430

Phe Ser Cys Ser Val Met His Glu Ala Leu His Asn His Tyr Thr Gln
        435             440             445

Lys Ser Leu Ser Leu Ser Pro Gly Lys
    450             455

<210> 14
<211> 214
<212> PRT
<213> Artificial Sequence

<220>
<223> RAM4lc

<400> 14

```
Asp Ile Gln Met Thr Gln Ser Pro Gly Thr Leu Ser Leu Ser Pro Gly
1               5               10              15

Glu Arg Ala Thr Leu Ser Cys Arg Ala Ser Gln Gly Val Ser Ser Ser
            20              25              30

Ser Leu Ala Trp Tyr Gln Gln Lys Pro Gly Gln Ala Pro Arg Leu Leu
            35              40              45

Ile Tyr Gly Thr Ser Ser Arg Ala Thr Gly Ile Pro Asp Arg Phe Ser
    50              55              60

Gly Ser Ala Ser Gly Thr Asp Phe Thr Leu Thr Ile Ser Arg Leu Gln
65              70              75              80

Pro Glu Asp Phe Ala Val Tyr Tyr Cys Gln Gln Tyr Gly Arg Ser Leu
            85              90              95

Thr Phe Gly Gly Gly Thr Lys Val Glu Ile Lys Arg Thr Val Ala Ala
            100             105             110

Pro Ser Val Phe Ile Phe Pro Pro Ser Asp Glu Gln Leu Lys Ser Gly
        115             120             125

Thr Ala Ser Val Val Cys Leu Leu Asn Asn Phe Tyr Pro Arg Glu Ala
    130             135             140

Lys Val Gln Trp Lys Val Asp Asn Ala Leu Gln Ser Gly Asn Ser Gln
145             150             155             160

Glu Ser Val Thr Glu Gln Asp Ser Lys Asp Ser Thr Tyr Ser Leu Ser
            165             170             175

Ser Thr Leu Thr Leu Ser Lys Ala Asp Tyr Glu Lys His Lys Val Tyr
        180             185             190

Ala Cys Glu Val Thr His Gln Gly Leu Ser Ser Pro Val Thr Lys Ser
        195             200             205

Phe Asn Arg Gly Glu Cys
    210
```